# EUROPÄISCHE PATENTSCHRIFT

(11) **EP 4 301 423 B1**
(45) Veröffentlichungstag und Bekanntmachung des Hinweises auf die Patenterteilung: **01.10.2025**
(21) Anmeldenummer: 22708280.7
(22) Anmeldetag: 28.02.2022
(51) Int. Cl.: A61L 9/04, A61L 9/12

(54) **AM KÖRPER EINES ANWENDERS TRAGBARE VORRICHTUNG ZUR ABGABE, VERDUNSTUNG UND/ODER VERDAMPFUNG EINES WIRKSTOFFES**
DEVICE THAT CAN BE WORN ON THE BODY OF A USER FOR DISPENSING, EVAPORATING AND/OR VAPORISING AN ACTIVE SUBSTANCE
DISPOSITIF POUVANT ÊTRE PORTÉ SUR LE CORPS D'UN UTILISATEUR DESTINÉ À LA DISTRIBUTION, À L'ÉVAPORATION ET/OU À LA VAPORISATION D'UNE SUBSTANCE ACTIVE

(30) Priorität: 01.03.2021 AT 501412021
(43) Veröffentlichungstag der Anmeldung: 10.01.2024
(73) Patentinhaber: Lindner, Manuel, 6250 Kundl (AT)
(72) Erfinder: Lindner, Manuel, 6250 Kundl (AT)
(74) Vertreter: Torggler & Hofmann Patentanwälte - Innsbruck
(86) Internationale Anmeldenummer: PCT/AT2022/060057
(87) Internationale Veröffentlichungsnummer: WO 2022/183229

(56) Entgegenhaltungen:
- WO-A1-2015/187523
- WO-A2-2011/019404
- JP-A- 2009 118 768

## Beschreibung

Die vorliegende Erfindung betrifft eine am Körper eines Anwenders tragbare Vorrichtung zur Abgabe, Verdunstung und/oder Verdampfung eines Wirkstoffes mit den Merkmalen des Anspruchs 1, eine Anordnung einer solchen tragbaren Vorrichtung mit zumindest einem Wirkstoff, sowie eine Verwendung eines Wirkstoffpads und/oder Trägergefäßes in einer solchen tragbaren Vorrichtung und/oder einer solchen Anordnung.

Unter Wirkstoffe können beispielsweise Duftstoffe, Parfums, zu inhalierende Medikamente, geruchsneutralisierende Stoffe und des Ähnlichen verstanden werden. Im Folgenden soll der Stand der Technik anhand von Duftstoffen umrissen werden. Analoges gilt allgemein für Wirkstoffe.

Gattungsgemäße, am Körper eines Anwenders tragbare Vorrichtungen zur Abgabe, Verdunstung und/oder Verdampfung eines Wirkstoffes umfassen
- wenigstens eine Speichervorrichtung zur Speicherung des Wirkstoffes, und
- wenigstens eine Ausbringöffnung, durch welche der Wirkstoff aus der wenigstens einen Speichervorrichtung an eine Umgebung der Vorrichtung entweichen kann.

So ist beispielsweise durch die US 1,732,028 A eine Vorrichtung bekannt, welche ein Gehäuse aufweist, in welchem ein Pad zur Aufnahme eines flüssigen Duftstoffes vorgesehen ist. Im Gehäuse sind Öffnungen angeordnet, durch welche der verdunstete oder verdampfte Duftstoff aus der Vorrichtung entweichen kann. Das Gehäuse kann über eine Anstecknadel an einem Anwender getragen werden. Nachteilig dabei ist jedoch, dass der Duftstoff bei einer frischen Befüllung einen intensiven Duft abgibt und sehr schnell diese gewünschte Intensivität verliert, da sich die gespeicherte Flüssigkeit des Duftstoffes verringert und somit auch die Abgabe des Duftstoffes durch die Vorrichtung über die Zeit (oder anders ausgedrückt: mit dem Beladungszustand) kontinuierlich verringert.

Ähnliche aus dem Stand der Technik bekannte Vorrichtungen sind beispielsweise durch die DE 19635528 A1, die DE 20 2007 004 622 U1 oder die WO 2005/123151 A2 bekannt.

Aus der WO 2015/187523 A1, der WO 2011/019404 A2, der JP 2009-118768 A und der GB 577605 A sind Vorrichtungen bekannt, welche ein Gehäuse aufweisen, in welchem ein Pad oder ein Trägergefäß zur Aufnahme eines flüssigen Duftstoffes vorgesehen ist. Der Duftstoff ist in diesen Vorrichtungen aktiv durch Antriebseinheiten förderbar. Nachteilig daran ist jedoch, dass stets ein kontinuierliches Fördern an Duftstoff an die Umgebung vonstattengeht, jedoch nicht auf die äußeren Einflüsse eingegangen werden kann. Somit wird bei besagten Vorrichtungen eine gleiche Menge an Duftstoff abgegeben, wenn ein Anwender verharrt - beispielsweise über längere Zeit sitzt - oder sich aktiv bewegt - beispielweise Sport betreibt - wodurch es entweder zu einer zu starken oder zu einer zu geringen Intensität der Duftstoffabgabe kommt.

Als nachteilig hat sich am Stand der Technik somit herausgestellt, dass ein Duftstoff und/oder Wirkstoff durch eine am Körper eines Anwenders tragbare Vorrichtung nicht abhängig von Umgebungseinflüssen abgegeben werden kann, da die Abgabe zumeist von einem Beladungszustand oder einer Leistung einer Antriebseinheit abhängig ist (wie bereits oben zum Stand der Technik ausgeführt).

Ein weiterer Nachteil des Standes der Technik ist, dass bekannte Vorrichtungen zumeist recht groß ausgebildet sind, was zu einem unangenehmen Tragegefühl des Anwenders und/oder einer optischen Beeinträchtigung der zumeist als Schmuck genutzten Vorrichtung führt.

Aufgabe der vorliegenden Erfindung ist es, eine am Körper eines Anwenders tragbare Vorrichtung zur Abgabe, Verdunstung und/oder Verdampfung eines Wirkstoffes bereitzustellen, mit welcher die zuvor beschriebenen Nachteile des Standes der Technik zumindest teilweise verbessert werden und/oder die Abgabe, Verdunstung und/oder Verdampfung des Wirkstoffes verbessert wird und/oder ein Tragegefühl eines Anwenders verbessert wird und/oder ein ästhetisches oder optisches Erscheinungsbild verbessert wird und/oder eine effizientere Nutzung des Wirkstoffes verbessert wird und/oder eine Abgabe eines Duftstoffes auf die Umgebung, vorzugsweise eine Bewegung eines Anwenders, angepasst werden kann.

Diese Aufgabe wird durch eine am Körper eines Anwenders tragbare Vorrichtung zur Abgabe, Verdunstung und/oder Verdampfung eines Wirkstoffes mit den Merkmalen des Anspruchs 1, mit einer Anordnung einer solcher Vorrichtung und zumindest einem Wirkstoff mit den Merkmalen des Anspruchs 13 und einer Verwendung eines Wirkstoffpads und/oder Trägergefäßes in einer solchen tragbaren Vorrichtung und/oder einer solchen Anordnung gelöst.

Erfindungsgemäß ist vorgesehen, dass eine am Körper eines Anwenders tragbare Vorrichtung zur Abgabe, Verdunstung und/oder Verdampfung eines Wirkstoffes folgendes umfasst:
- wenigstens eine Speichervorrichtung zur Speicherung des Wirkstoffes,
- wenigstens einer Ausbringvorrichtung, durch welche der Wirkstoff aus der wenigstens einen Speichervorrichtung in eine Umgebung der Vorrichtung entweichen kann,
wobei eine mit bewegbaren, mechanischen Mitteln vorgesehene Fördervorrichtung zur Erzeugung einer Luftbewegung vorgesehen ist, durch welche Wirkstoff durch die wenigstens eine Ausbringöffnung förderbar ist, wobei die bewegbaren mechanischen Mittel durch wenigstens einen Rotor umgesetzt sind, welche um wenigstens eine Rotationsachse drehbar gelagert ist.

Durch das Vorsehen bewegbarer, mechanischer Mittel als Fördervorrichtung wird die Abgabe, Verdunstung und/oder Verdampfung eines Wirkstoffes gefördert, indem eine Luftzirkulation an der wenigstes einen Speichervorrichtung gefördert oder hervorgerufen wird.

Das Ausbringen des Wirkstoffes wird durch die bewegbaren, mechanischen Mittel als Fördervorrichtung und eine dadurch hervorgerufene Luftzirkulation gefördert, wodurch eine kontinuierliche Ausbringung des Wirkstoffes hervorgerufen wird - unabhängig von einem Speicherzustand der wenigstens einen Speichervorrichtung.

Des Weiteren kann im Vergleich zum Stand der Technik durch eine Fördervorrichtung der Effekt der Abgabe, Verdunstung und/oder Verdampfung des Wirkstoffes vergrößert werden, sodass der Wirkstoff effektiver an eine Umgebung der Vorrichtung freigesetzt werden kann.

Weiters ist es nicht mehr erforderlich, größere Speichervorrichtungen vorzusehen, um einen gewünschten Effekt zu erzielen, da auch durch kleinere Speichervorrichtungen durch die Fördervorrichtung eine ausreichend große Abgabe, Verdunstung und/oder Verdampfung des Wirkstoffes hervorgerufen werden kann.

Durch Vorsehen wenigstens eines Rotors als Fördervorrichtung wird somit die Fördervorrichtung nur durch die Bewegung des Anwenders angetrieben, womit eine Förderung des Duftstoffes nur bei einer Bewegung der tragbaren Vorrichtung durch den Anwender erfolgt.

Dies hat den wesentlichen Vorteil gegenüber dem Stand der Technik, dass die Intensität der Duftstoffabgabe nicht von einem Füllstand oder einer Antriebsleistung einer Antriebseinheit abhängt, sondern individuell an die Bedürfnisse des Anwenders angepasst sind.

So wird bei einer starken Bewegung der tragbaren Vorrichtung - wie sie beispielsweise auftritt, wenn ein Anwender Sport betreibt - eine erhöhte Intensität an Duftstoff durch die höhere Bewegung des Rotors (und somit der Fördervorrichtung) abgegeben, wobei unangenehme Gerüche durch die erhöhte Bewegung des Anwenders (wie der Geruch von Schweiß) überdeckt werden können.

Andererseits wird die Intensität der Abgabe von Duftstoff reduziert, wenn die Bewegung der tragbaren Vorrichtung und somit der Antrieb der Fördervorrichtung durch den Rotor reduziert wird, wie es beispielsweise der Fall ist, wenn ein Anwender längere Zeit (wie beispielweise bei der Arbeit an einem Schreibtisch) sitzt. Durch diese reduzierte Abgabe von Duftstoff kann eine zu hohe auftretende Konzentration und/oder Intensität an Duftstoff, welche zumeist einem Anwender als unangenehm auffallen würde, vermieden werden.

Weiters ist durch die Anwendung wenigstens einen Rotors zur Förderung der Fördervorrichtung eine rein mechanische Lösung geschaffen, welche keine zusätzlichen Antriebseinheiten durch Motoren oder Getriebe erforderlich macht und somit sehr wartungsfreundlich ist.

Im Sinne des vorliegenden Dokumentes ist ein Rotor so zu verstehen, dass der Rotor wenigstens eine Unwucht bezüglich seiner Rotationsachse (bespielweise eine Gewichtsasymmetrie) aufweist, wobei durch Bewegung der Vorrichtung über die wenigstens eine Unwucht (sozusagen passiv über die Bewegung der gesamten Vorrichtung) eine Rotationsbewegung des Rotors hervorgerufen werden kann. Die Unwucht des Rotors kann durch eine konstruktive Ausgestaltung und/oder eine geeignete Materialwahl zur Umsetzung einer Gewichtsasymmetrie hinsichtlich der Rotationsachse des Rotors umgesetzt werden.

Der wenigstens eine Rotor kann beispielsweise als asymmetrische Schwungmaße ausgebildet sein oder eine solche aufweisen.

Die Fördervorrichtung kann im Sinne der vorliegenden Erfindung in fluidtechnischer Verbindung mit der wenigstens einen Speichervorrichtung und/oder der wenigstens einen Ausbringöffnung stehen.

Die Fördervorrichtung kann dazu ausgebildet sein, eine Luftbewegung, Luftströmung und/oder Luftzirkulation an oder in der wenigstens einen Speichervorrichtung hervorzurufen und/oder zu fördern.

Als Wirkstoff können beispielsweise Duftstoffe, Parfums, Medikamente, Inhalate, geruchsneutralisierende Stoffe oder der Ähnlichen ihre Anwendung finden.

Eine erfindungsgemäße Vorrichtung kann auch in bereits bekannten Ausführungsvarianten des Standes der Technik, wie beispielsweise in der Beschreibungseinleitung beschrieben, ihren Einsatz finden und nachträglich installiert werden.

Vorteilhafte Ausführungsformen der Erfindung sind anhand der abhängigen Ansprüche definiert.

Es kann beispielweise vorgesehen sein, dass der wenigstens eine Rotor als Zentralrotor, dezentraler Rotor und/oder Microrotor umgesetzt ist.

Es kann vorgesehen sein, dass die Fördervorrichtung dazu ausgebildet ist, vorzugsweise direkt, die wenigstens eine Speichervorrichtung zur Speicherung des Wirkstoffes aufzunehmen.

Durch die, vorzugsweise direkte, Aufnahme der wenigstens einen Speichervorrichtung durch die Fördervorrichtung kann es vorgesehen sein, dass eine Bewegung der Fördervorrichtung direkt auf die wenigstens eine Speichervorrichtung umgesetzt wird.

So könnte es beispielsweise vorgesehen sein, dass die wenigstens eine Speichervorrichtung direkt auf dem wenigstens einen Rotor und/oder der Fördervorrichtung lösbar gelagert und/oder drehfest zur Rotationsachse verbunden ist, wodurch sich eine, vorzugsweise Rotations-, Bewegung des wenigstens einen Rotors und/oder der Fördervorrichtung direkt auf die wenigstens eine Speichervorrichtung überträgt, wobei die wenigstens eine Speichervorrichtung zusammen mit dem wenigstens einen Rotor und/oder der Fördervorrichtung bewegbar ist.

Es kann vorgesehen sein, dass die wenigstens eine Speichervorrichtung mit der Fördervorrichtung durch wenigstens eine Verbindungsvorrichtung, vorzugsweise drehfest um die Rotationsachse, lösbar befestigbar ist. Die wenigstens eine Verbindungsvorrichtung kann beispielsweise als formschlüssige Verbindung, kraftschlüssige Verbindung und/oder über Magnetelemente ausgebildet sein.

Es ist vorgesehen, dass der wenigstens eine Rotor wenigstens eine Kippkante aufweist, welche Kippkante dazu ausgebildet ist, dass die wenigstens eine Speichervorrichtung zur Entnahme aus der tragbaren Vorrichtung bei einer asymmetrischen Krafteinwirkung auf die wenigstens eine Speichervorrichtung über die Kippkante verkippt.

Folglich kann es vorgesehen sein, dass beispielsweise eine bereits vorgesehene Asymmetrie des wenigstens einen Rotors zusätzlich durch eine Kippkante versehen wird, welche Kippkante dazu genutzt werden kann, die wenigstens eine Speichervorrichtung gegenüber dem wenigstens einen Rotor und/oder der tragbaren Vorrichtung bei einer Entnahme oder einer Zufuhr der wenigstens einen Speichervorrichtung zu verkippen, um dem Anwender eine einfachere Entnahme und/oder Zufuhr der wenigstens einen Speichervorrichtung zu ermöglichen.

Eine entsprechende Kippkante kann Unterbrechungen aufweisen, als durchgehende Kante ausgebildet sein, durch eine abgerundete Kante gebildet werden und/oder entlang ihrer Längserstreckung eine von einer Geraden abweichende Form aufweisen.

Vorzugsweise kann vorgesehen sein, dass der wenigstens eine Rotor die wenigstens eine Fördervorrichtung ausbildet und der wenigstens eine Rotor die wenigstens eine Verbindungsvorrichtung und/oder die wenigstens eine Kippkante aufweist und/oder ausbildet.

Durch die Ausbildung des wenigstens einen Rotors als Fördervorrichtung, Verbindungsvorrichtung und/oder Kippkante kann eine Vielzahl an Komponenten eingespart werden, wodurch sich die Komplexität, der Fertigungsaufwand, sowie der Wartungsaufwand der Vorrichtung verringert.

Es kann vorgesehen sein, dass die wenigstens eine Speichervorrichtung ein Gehäuseelement aufweist, welches Gehäuseelement dazu ausgebildet ist, den Wirkstoff aufzunehmen.

Es kann vorgesehen sein, dass das Gehäuseelement ein- oder mehrteilig ausgebildet ist. Bei einer mehrteiligen Ausgestaltung des Gehäuseelementes kann eine Verbindung der Gehäuseelemente durch Kraft-, Form- oder Stoffschluss erfolgen.

Vorzugsweise ist vorgesehen, dass die wenigstens eine Ausbringöffnung zumindest eine Öffnung - vorzugsweise eine Bohrung und/oder ein Durchgangsloch - im Gehäuseelement aufweist, welche das Innere des Gehäuseelements mit der Umgebung verbindet.

Es kann vorgesehen sein, dass das Gehäuseelement wenigstens einen transparenten Teilbereich aufweist - vorzugsweise in Form eines transparenten Deckels - mittels welchem das Innere des Gehäuseelementes durch einen Anwender ersichtlich ist.

So kann beispielsweise im Inneren des Gehäuseelementes auch ein weiteres, optisch ansprechendes Element seine Platzierung finden (wie beispielsweise eine Uhrzeitanzeige, ein Logo, ein Schmuckelement oder Edelsteine), welche durch einen teilweise transparenten Bereich des Gehäuses durch einen Anwender ersichtlich ist, jedoch dennoch durch das Gehäuse vor Umgebungseinflüssen, vor Beschädigungen oder Verschmutzung geschützt werden kann.

Besondres bevorzugt kann vorgesehen sein, dass ein optisch ansprechendes Element (wie beispielsweise eine Uhrzeitanzeige, ein Logo, ein Schmuckelement oder Edelsteine) mit dem wenigstens einen Rotor verbunden ist, sodass das optisch ansprechende Element unter Zuhilfenahme des wenigstens einen Rotors und der Schwerkraft in jeder Lage der tragbaren Vorrichtung horizontal ausgerichtet wird und für einen Anwender stets bequem ablesbar ist.

Beispielsweise kann es auch vorgesehen sein, dass das Gehäuseelement wenigstens eine (vergrößerte) Austrittsöffnung aufweist, mittels welcher das Innere des Gehäuseelementes durch einen Anwender ersichtlich ist.

Es kann vorgesehen sein, dass die Fördervorrichtung im Inneren des Gehäuseelementes angeordnet ist.

Bei einer Anordnung des Förderelementes im Inneren des Gehäuses ergibt sich die positive Eigenschaft, dass das Gehäuseelement die Fördervorrichtung von äußeren Einflüssen, wie Verschmutzungen oder Beschädigungen schützen kann, sodass eine einwandfreie Funktionalität des Förderelementes gewährleistet werden kann. Folglich können Wartungs-, Reinigungs-. oder Reparaturarbeiten reduziert werden.

Es kann vorgesehen sein, dass die Fördervorrichtung wenigstens eine aktive Antriebseinheit - vorzugsweise einen Elektroantrieb - aufweist, welche dazu ausgebildet ist, eine Bewegung der Fördervorrichtung, vorzugsweise des wenigstens einen Rotors, zu verstärken. Als aktive Antriebseinheiten können Antriebseinheiten verstanden werden, welche durch einen vorliegenden Energiespeicher (beispielsweise eine Batterie) gespeist werden.

Vorzugsweise ist vorgesehen, dass die Fördervorrichtung wenigstens eine passive Antriebseinheit - vorzugsweise ein Unruh-Spirale-Schwingsystem, ein Spiralfedersystem und/oder ein Schwingungssystem - aufweist, welche dazu ausgebildet ist, eine Bewegung der Fördervorrichtung, vorzugsweise des wenigstens einen Rotors, zu verstärken. Solche Unruh-Spirale-Schwingsysteme sind durch den Stand der Technik auch weitläufig unter dem Namen eines Uhrwerkes bekannt. Als passive Antriebseinheit kann verstanden werden, dass jegliche Bewegung der Vorrichtung als Antrieb der Fördervorrichtung genutzt werden kann, welche beispielsweise auch durch ein Unruh-Spirale-Schwingsystem, ein Spiralfedersystem und/oder ein Schwingungssystem zwischengespeichert werden könnte.

Eine aktive oder passive Antriebseinheit ist im Sinne des vorliegenden Dokumentes jedoch lediglich als unterstützende Antriebseinheit des wenigstens einen Rotors zu verstehen, welche eine Bewegung des wenigstens einen Rotors verstärkt und/oder fördert, jedoch keine eigenständige Bewegung umsetzt.

Es kann vorgesehen sein, dass die Vorrichtung zumindest als Teil einer tragbaren Uhr - vorzugsweise einer Armbanduhr - und/oder eines Schmuckstücks ausgebildet ist.

So kann es beispielsweise vorgesehen sein, dass als aktive Antriebseinheit der Fördervorrichtung die Antriebseinheit einer Armbanduhr dient (beispielsweise ein Elektroantrieb oder eine Unruhwerk).

Vorzugsweise kann vorgesehen sein, dass die Vorrichtung wenigstens eine Anzeigevorrichtung aufweist, wobei die wenigstens eine Anzeigevorrichtung vorzugsweise
- eine Uhrzeitanzeige und/oder
- eine Datumsanzeige und/oder
- eine Gravur und/oder
- eine Werbefläche und/oder
- ein Display und/oder
- ein NFT (Non-Fungible-Token) -Kunstwerk und/oder
- einen QR-Code und/oder
- ein Symbol
aufweist.

Diese Anzeigevorrichtung kann beispielsweise auch im Inneren eines Gehäuses angeordnet sein, welches durch ein teilweise transparent ausgebildetes Gehäuseelement für einen den Anwender sichtbar ist. Jedoch ist auch die Anordnung der Anzeigevorrichtung an einer äußeren Oberfläche der Vorrichtung oder eines Gehäuseelementes durchaus denkbar.

So kann es beispielsweise vorgesehen sein, dass durch eine Anzeigevorrichtung eine Marke und/oder ein Produktname des Wirkstoffes, welcher in der wenigstens einen Speichervorrichtung angeordnet ist, ersichtlich ist. Durch die Bewegung des wenigstens einen Rotors (welcher gegebenenfalls auch die Anzeigevorrichtung bewegt) kann zusätzlich die Aufmerksamkeit eines Anwenders über den transparenten Teilbereich auf die Anzeigevorrichtung gelenkt werden.

Vorzugsweise ist vorgesehen, dass die Anzeigevorrichtung bewegungsschlüssig mit dem wenigstens einen Rotor verbindbar ist, sodass die Anzeigevorrichtung unter Zuhilfenahme des wenigstens einen Rotors und der Schwerkraft in jeder Lage der tragbaren Vorrichtung horizontal ausgerichtet werden kann und für einen Anwender stets bequem ablesbar und/oder ersichtlich ist.

Vorzugsweise kann vorgesehen sein, dass die wenigstens eine Vorrichtung eine individuell anpassbare Anzeigevorrichtung, vorzugsweis Gravur, aufweist.

So könnten beispielsweise an der Vorrichtung an einer sichtbaren Fläche eine individuell anpassbare Anzeigevorrichtung, wie beispielsweise ein Public Key, Private Key oder auch ein Seed Key (hinsichtlich einer Kryptowährung), angeordnet sein. Jedoch wäre auch durchaus denkbar, dass beispielsweise an einer sichtbaren Fläche der Vorrichtung ein Symbol einer Kryptowährung - beispielweise das Bitcoin Zeichen - oder ein Public Key angeordnet ist und an einer von außen nicht ersichtlichen Fläche der Vorrichtung (beispielsweise einer Unterseite der Speichervorrichtung, welche nur in einem demontierten Zustand der Speichervorrichtung ersichtlich ist) ein Private Key oder auch ein Seed Key angeordnet ist.

Es kann auch vorgesehen ein, dass die Vorrichtung eine Aufnahme für eine Anzeigevorrichtung aufweist, wobei beispielsweise in der Speichervorrichtung eine Aufnahme für eine Anzeigevorrichtung, vorzugsweise ein Blatt Papier, vorgesehen sein kann, um individuelle Botschaften, Codes oder Passwörter eines Anwenders aufnehmen zu können.

Es kann auch vorgesehen ein, dass die Vorrichtung eine Aufnahmevorrichtung zur Aufnahme einer Wallet (beispielsweise eines Cyberwallet, Kryptowallet, ...) - beispielsweise einer Hardware-Wallet - aufweist.

Es kann vorgesehen sein, dass die Vorrichtung eine Haltevorrichtung - vorzugsweise umfassend ein Armband und/oder eine Anstecknadel und/oder eine Öse und/oder Klemmvorrichtung und/oder eine Magnethalterung - aufweist, wobei die Vorrichtung mittels der Haltevorrichtung an einem Körper eines Anwenders befestigbar und oder tragbar ist.

Durch die Haltevorrichtung kann die Vorrichtung somit direkt am Körper eines Anwenders (beispielsweise durch ein Armband) befestigt oder getragen werden oder indirekt an einem Kleidungsteil des Anwenders (beispielsweise durch eine Anstecknadel und/oder eine Öse) befestigt oder getragen werden. Auch die Verwendung der Vorrichtung als Ohrring (über eine Anstecknadel) ist durchaus vorstellbar. Vorzugsweise kann auch vorgesehen sein, dass die Vorrichtung durch die Haltevorrichtung mit einer Tasche und/oder einem Rucksack verbunden werden kann.

Es kann beispielsweise sein, dass die Haltevorrichtung als Magnethalterung ausgebildet ist, wobei die Vorrichtung durch die magnetisch ausgebildete Haltevorrichtung an entsprechend magnetisierbaren Gegenständen anhaften kann.

Es kann vorgesehen sein, dass die Abgabevorrichtung eine zylindrische Außenform aufweist, dessen Durchmesser vorzugsweise mit einer geringeren Abmessung ausgebildet ist als eine Längserstreckung. So kann beispielsweise das Verhältnis einer Längserstreckung zum Durchmesser in einer Größenordnung von 0,5 bis 0,1 - vorzugsweise in einem Verhältnis von 0,4 bis 0,15, besonders bevorzugt in einem Verhältnis von 0,3 - liegen.

Alternativ sind jedoch auch eckige, rechteckige, polygonale oder kugelförmige Außenformen (oder jegliche andere vorstellbare Geometrie) der Abgabevorrichtung vorstellbar, wobei die Verhältnisse der Außenformen zu einer Längserstreckung denen der oben angeführten entsprechen können oder auch abweichen können.

Vorzugsweise ist vorgesehen, dass der wenigstens eine Rotor quer zur Rotationsachse einen Vorsprung und/oder eine Strömungsleiteinrichtung und/oder eine Finne zur Förderung der Luftbewegung aufweist. So kann beispielsweise ein Vorsprung und/oder eine Strömungsleiteinrichtung und/oder eine Finne am Rotor vorgesehen sein, welche ähnlich zu einem Ventilator eine Luftbewegung hervorrufen oder begünstigen kann.

Alternativ oder zusätzlich können auch jegliche andere eine Luftbewegung intensivierende oder erzeugende Geometrien vorgesehen sein. So können Geometrien, die durch ihre Ausrichtung und Bewegung eine Luftbewegung erzeugen, vorgesehen sein.

Die Luftbewegung kann in eine definierte Richtung ausgerichtet sein (wie beispielsweise durch eine Turbine) oder eine diffuse Luftverwirbelung erzeugen.

Weiters wird Schutz begehrt für eine Anordnung einer tragbaren Vorrichtung gemäß eines Ausführungsbeispiels der Erfindung und zumindest einem Wirkstoff, vorzugsweise ein Parfum, wobei der zumindest eine Wirkstoff in der wenigstens einen Speichervorrichtung der Vorrichtung angeordnet ist.

Vorzugsweise kann vorgesehen sein, dass der zumindest eine Wirkstoff
- in einem Wirkstoffpad gespeichert ist, welches aus einem den zumindest einen Wirkstoff aufnehmenden Werkstoff ausgebildet ist, wobei der zumindest eine Wirkstoff durch Verdunstung, Verdampfung oder mechanische Einflüsse vom Wirkstoffpad abgebbar ist und/oder
- in einem Trägergefäß zur flüssigen, festen und/oder gasförmigen Aufnahme des zumindest einen Wirkstoffes gespeichert ist, wobei der zumindest eine Wirkstoff durch eine Öffnung im Trägergefäß abgebbar ist
wobei das Wirkstoffpad und/oder das Trägergefäß in der wenigstens einen Speichervorrichtung angeordnet ist.

Es ist auch eine Ausgestaltung denkbar, bei welcher der zumindest eine Wirkstoff - vorzugsweise in flüssiger Form - direkt in der wenigstens einen Speichervorrichtung vorgesehen ist.

Auch hochviskose Wirkstoffe oder feste, einen Wirkstoff abgebende Substrate oder Substanzen zu Aufnahme eines Wirkstoffes sind durchaus denkbar und im Zuge einer Ausführungsvariante anwendbar.

In Frage kommende Wirkstoffpads sind durch Werkstoffe ausgebildet, welche den zumindest einen Wirkstoff aufnehmen können, wobei der zumindest eine Wirkstoff durch das Wirkstoffpad in Poren aufgesaugt oder getragen wird. Jedoch sind auch Werkstoffe denkbar, welche den Wirkstoff durch eine chemische Reaktion oder ein physikalisches Einwirken aufnehmen können und auch in der Lage sind, diesen wieder abzugeben.

Weiters wird Schutz begehrt für eine Verwendung
- eines Wirkstoffpads, in welchem zumindest ein Wirkstoff gespeichert ist, wobei der zumindest eine Wirkstoff durch Verdunstung, Verdampfung und/oder mechanische Einflüsse vom Wirkstoffpad abgebbar ist, und/oder
- eines Trägergefäßes, welches dazu ausgebildet ist, zumindest einen flüssigen, festen und/oder gasförmigen Wirkstoffes aufzunehmen, wobei der zumindest eine Wirkstoff durch eine Öffnung im Trägergefäß abgebbar ist,
in einer erfindungsgemäßen tragbaren Vorrichtung und/oder einer erfindungsgemäßen Anordnung.

Weitere Beispiele, Vorteile und Einzelheiten der Erfindung sind in den Figuren und der nachstehenden Figurenbeschreibung dargestellt. Darin zeigen:
- Fig. 1: ein erstes Ausführungsbeispiel einer Vorrichtung,
- Fig. 2: eine Seitenansicht der Fig. 1,
- Fig. 3: einen Schnitt quer durch Fig. 2,
- Fig. 4: ein zweites Ausführungsbeispiel einer Vorrichtung in einer ersten Stellung,
- Fig. 5: den in Fig. 4 gekennzeichneten Schnitt A-A,
- Fig. 6: ein zweites Ausführungsbeispiel der Fig. 4 in einer zweiten Stellung,
- Fig. 7: ein drittes Ausführungsbeispiel einer Vorrichtung,
- Fig. 8: ein weiteres Ausführungsbeispiel einer Vorrichtung,
- Fig. 9-11: unterschiedliche elektrische Antriebseinheiten für eine Fördervorrichtung,
- Fig. 12, 13: eine passive Antriebseinheit für eine Fördervorrichtung,
- Fig. 14, 15: eine weitere passive Antriebseinheit für eine Fördervorrichtung,
- Fig. 16-18: verschiedene Ausführungsvarianten einer Lagerung einer Fördervorrichtung,
- Fig. 19: verschiedene Ausführungsvarianten einer Speichervorrichtung,
- Fig. 20-23: verschiedene Ausführungsformen einer Speichervorrichtung,
- Fig. 24-26: verschiedene Ausführungsvarianten einer Anordnung eines Trägergefäßes in einer Vorrichtung,
- Fig. 27-30: verschiedene Ausführungsvarianten einer Haltevorrichtung einer Vorrichtung,
- Fig. 31-33: verschiedene Ausführungsvarianten einer Anzeigevorrichtung einer Vorrichtung,
- Fig. 34, 35: die Anwendung einer tragbaren Vorrichtung an einem Körper eines Anwenders,
- Fig. 36-42: ein weiteres Ausführungsbeispiel einer Vorrichtung, und
- Fig. 43-49: ein weiteres Ausführungsbeispiel einer Vorrichtung.

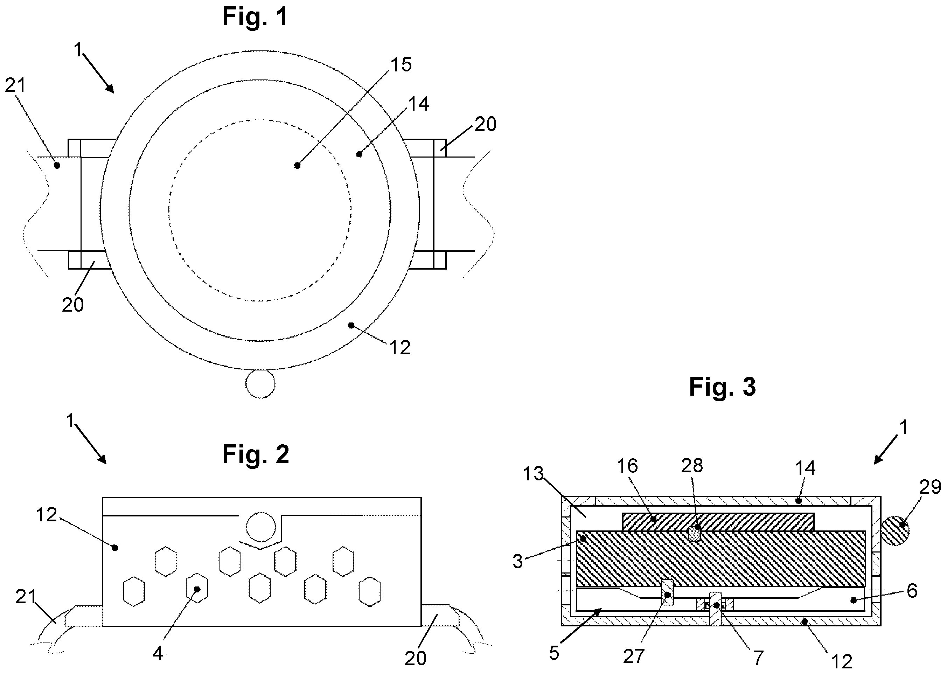

Fig. 1 zeigt ein erstes Ausführungsbeispiel einer erfindungsgemäßen, am Körper eines Anwenders tragbaren Vorrichtung 1.

Dabei zeigt Fig. 1 eine Draufsicht auf die Vorrichtung 1, Fig. 2 eine Seitenansicht der Fig. 1 und Fig. 3 einen seitlichen Schnitt durch die Fig. 2.

Das Ausführungsbeispiel der Figuren 1 bis 3 einer Ausführungsvariante einer erfindungsgemäßen Vorrichtung 1 weist ein Gehäuseelement 12 auf, welches zylindrisch ausgebildet ist und ähnliche Abmessungen wie eine Armbanduhr aufweist.

Dieses Gehäuseelement 12 kann über die Haltevorrichtungen 20 mit einem Armband 21 am Körper eines Anwenders getragen werden.

Das Gehäuseelement 12 der Vorrichtung 1, welches zylinderförmig ausgebildet ist, wird durch einen Deckel 14 verschlossen, wobei der Deckel 14 einen transparenten Bereich 15 aufweist, durch welchen transparenten Bereich 15 ein Anwender das Innere 13 des Gehäuseelementes 12 einsehen kann. Dieser transparente Teilbereich 15 kann beispielsweise einen Glaswerkstoff oder auch andere transparente Werkstoffe ausgebildet sein.

Alternativ könnte dieser transparente Teilbereich 15 des Gehäuseelementes 12 auch durch eine Öffnung ausgebildet sein, wobei durch die Öffnung (anstelle des transparenten Teilbereiches 15) das Innere 13 des Gehäuseelementes 12 durch einen Anwender ersichtlich ist.

Der Deckel 14 des Gehäuseelementes 12 ist öffenbar ausgestaltet, wodurch das Innere 13 des Gehäuseelementes 12 zugänglich wird.

Das Innere 13 des Gehäuseelementes 12 wird durch die Austrittsöffnungen 4 mit der Umgebung der Vorrichtung 1 fluidtechnisch verbunden.

Im Inneren 13 des Gehäuseelementes 12 der Vorrichtung 1 ist die Fördervorrichtung 5 angeordnet.

In diesem spezifischen Ausführungsbeispiel der Vorrichtung 1 verfügt die Fördervorrichtung 5 über einen auf einer Rotationsachse 7 gelagerten Rotor 6, welcher dazu ausgebildet ist, eine Rotationsbewegung um die Rotationsachse 7 auszuführen.

Auf dem Rotor 6 ist durch das Lager 27 bewegungsschlüssig die Speichervorrichtung 3 (welche zur Aufnahme eines Wirkstoffes 2 ausgebildet ist) angeordnet.

Durch diese bewegungsschlüssige Anordnung der Speichervorrichtung 3 auf der Fördervorrichtung 5 (genauer gesagt: dem Rotor 6) wird die Speichervorrichtung 3 ebenfalls durch die Rotationsbewegung des Rotors 6 in Bewegung versetzt, wodurch die Fördervorrichtung 5 die Speichervorrichtung 3 gegenüber einer Umgebungsluft bewegt und somit eine Abgabe, Verdunstung und/oder Verdampfung eines in der Speichervorrichtung 3 gespeicherten Wirkstoffes 2 fördert.

Bezüglich möglicher Ausführungsvarianten der Speichervorrichtung 3 zur Aufnahme eines Wirkstoffes 2 wird auf die folgenden Figuren verwiesen.

Des Weiteren ist über das Lager 28 eine Anzeigevorrichtung 16 mit der Speichervorrichtung 3 und dem Rotor 6 verbunden, wobei auf der Anzeigevorrichtung eine Werbefläche 18, eine Gravur oder Ähnliches angeordnet werden kann, welche durch den transparent ausgebildeten Teilbereich 15 des Deckels 14 durch den Anwender ersichtlich ist.

Durch die Verbindung der Anzeigevorrichtung 16 mit dem Rotor 6 ist es somit möglich, unabhängig von der Lage der Vorrichtung 12 die Anzeigevorrichtung 16 horizontal auszurichten, da sich der Rotor 6 stets über die Unwucht und/oder Gewichtsasymmetrie gegenüber der Rotationsachse 7 durch die Gewichtskraft und/oder Erdanziehung ausrichtet.

Weiters ist ein Mechanismus 29 am Gehäuseelement 12 vorgesehen, wobei durch den Mechanismus 29 ein gegebenenfalls als Antriebseinheit vorgesehenes Uhrwerk aufgezogen oder eingestellt werden könnte oder der Mechanismus 29 möglicherweise zum Öffnen des Gehäuseelementes 12 dienen könnte.

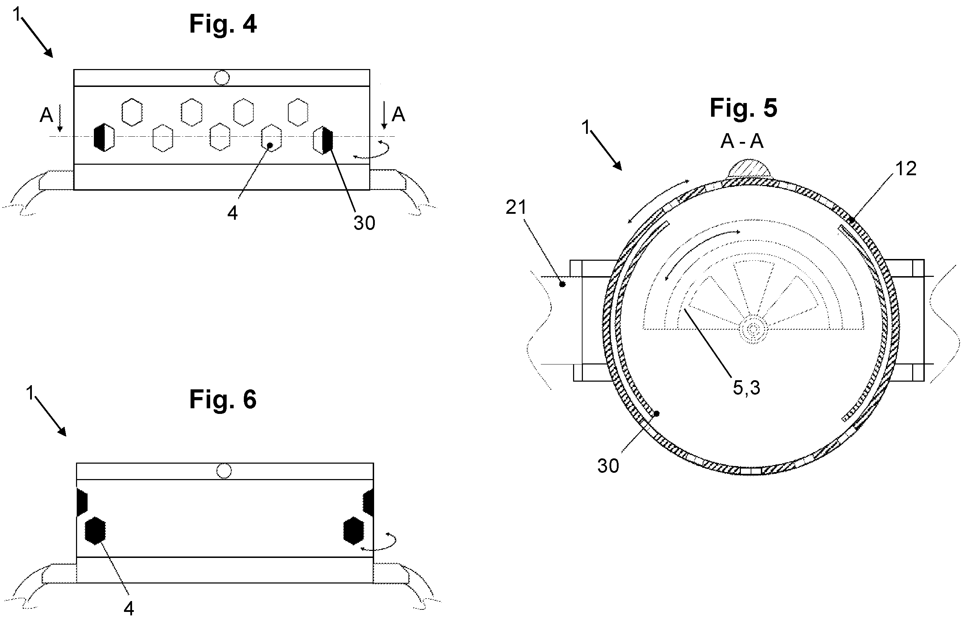

Fig. 4 zeigt ein zweites Ausführungsbeispiel einer erfindungsgemäßen Vorrichtung 1 in einer Seitenansicht in einer ersten Stellung. Fig. 5 zeigt das Ausführungsbeispiel der Fig. 4 im gekennzeichneten Schnitt A-A, welcher in Fig. 4 angedeutet ist. Fig. 6 zeigt das zweite Ausführungsbeispiel der Fig. 4 in einer zweiten Stellung.

Dieses zweite erfindungsgemäße Ausführungsbeispiel einer Vorrichtung 1 der Figuren 4 bis 6 weist wiederum im Inneren 13 des Gehäuseelementes 12 eine Fördervorrichtung 5 und eine Speichervorrichtung 3 auf, welche ähnlich wie in den Figuren 1 bis 3 ausgeführt sein können. Diese sind aus Gründen der Übersichtlichkeit in den Figuren 4 bis 6 nur angedeutet.

Zusätzlich zur Ausführungsvariante der Figuren 1 bis 3 weist jedoch das Ausführungsbeispiel der Figuren 4 bis 6 ein Verschlusselement 30 auf, welches im Inneren 13 des Gehäuseelementes 12 rotierbar angeordnet ist.

Durch dieses Verschlusselement 30 lassen sich einzelne Ausbringöffnungen 4 des Gehäuseelementes 12 verdecken, wodurch ein Austreten des Wirkstoffs 2 aus dem Inneren 13 des Gehäuseelements 12 verhindert werden kann, wodurch die Möglichkeit geschaffen wird, die Intensität eines austretenden Wirkstoffes 2 aus dem Gehäuseelement 12 zu steuern.

Dies ist ersichtlich durch die zwei Stellungen des Verschlusselementes 30 in der Vorrichtung 1, wie durch die Figuren 4 und 6 dargestellt ist.

So sind in Fig. 4 nur wenige seitliche Ausbringöffnungen 4 durch das Verschlusselement 30 verdeckt und ein möglichst großer Austritt des Wirkstoffes 2 aus dem Inneren 13 des Gehäuseelementes 12 wird ermöglicht.

Durch Rotation des Verschlusselements 30 können - wie in Fig. 6 dargestellt ist - mehrere Ausbringöffnungen 4 verdeckt werden, sodass nur ein gehemmter, kontrollierter oder vollständiger Austritt des Wirkstoffs 2 aus dem Inneren 13 des Gehäuseelementes 12 ermöglicht wird.

Es könnte jedoch auch vorgesehen sein, dass das Verschlusselement 30 gegenüber der Haltevorrichtung 20 der Vorrichtung fest ausgebildet ist und das Gehäuseelement 12 gegenüber dem Verschlusselement 30 rotierbar, verschiebbar und/oder arretierbar gelagert ist.

Jedoch ist es auch durchaus denkbar, das Verschlusselement 30 rotierend anzuordnen, sodass das Verschlusselement 30 zur Unterstützung Fördervorrichtung 5 genutzt werden kann.

So kann durch das Verschlusselement 30 durch Rotation eine Luftverwirbelung erzeugt werden, welche die Abgabe, Verdunstung und/oder Verdampfung des Wirkstoffes 2 im Inneren 13 des Gehäuseelementes 12 begünstigt und eine Förderung des Wirkstoffes 2 aus dem Inneren 13 des Gehäuseelementes 12 durch die Ausbringöffnungen 4 fördert.

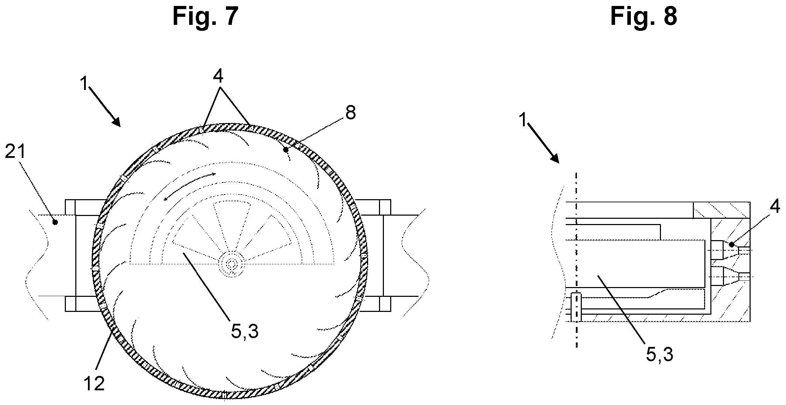

Fig. 7 zeigt ein drittes Ausführungsbeispiel einer erfindungsgemäßen Vorrichtung 1 in einem Schnitt, wobei wiederum in einem Inneren 13 eines Gehäuseelementes 12 eine Speichervorrichtung 3 und eine Fördervorrichtung 5 angeordnet sind, welche gemäß dem Ausführungsbeispiel der vorhergehenden Figuren ausgebildet sein kann und in dieser Figur nur schematisch dargestellt ist, um eine bessere Übersichtlichkeit zu generieren.

Das Gehäuseelement 12 der Fig. 7 weist jedoch zusätzlich Leiteinrichtungen 8 auf, welche eine Luftströmung aus dem Inneren 13 aus dem Gehäuseelementes 12 durch die Ausbringöffnungen 4 fördert und somit das Ausbringen eines Wirkstoffes 2 aus dem Inneren 13 des Gehäuseelementes 12 optimiert.

Fig. 8 zeigt ein weiteres Ausführungsbeispiel einer erfindungsgemäßen Vorrichtung 1, wobei das Innere 13 des Gehäuseelementes 12 wiederum mit der Fördervorrichtung 5 und der Speichervorrichtung 3 ähnlich den vorangehenden Ausführungsbeispielen ausgestaltet werden kann, jedoch die Ausbringöffnungen 4 im Gehäuseelement 12 mit einem sich entlang ihrer Erstreckung variierenden Querschnitt ausgebildet sind.

Die in der Ausführungsvariante der Fig. 8 dargestellten Ausbringöffnungen 4 weisen eine düsenförmige Ausgestaltung auf, welche dazu genutzt werden kann, um ausströmende Luft aus dem Inneren 13 des Gehäuseelementes 12 und den damit transportierten Wirkstoff 2 strömungstechnisch zu optimieren.

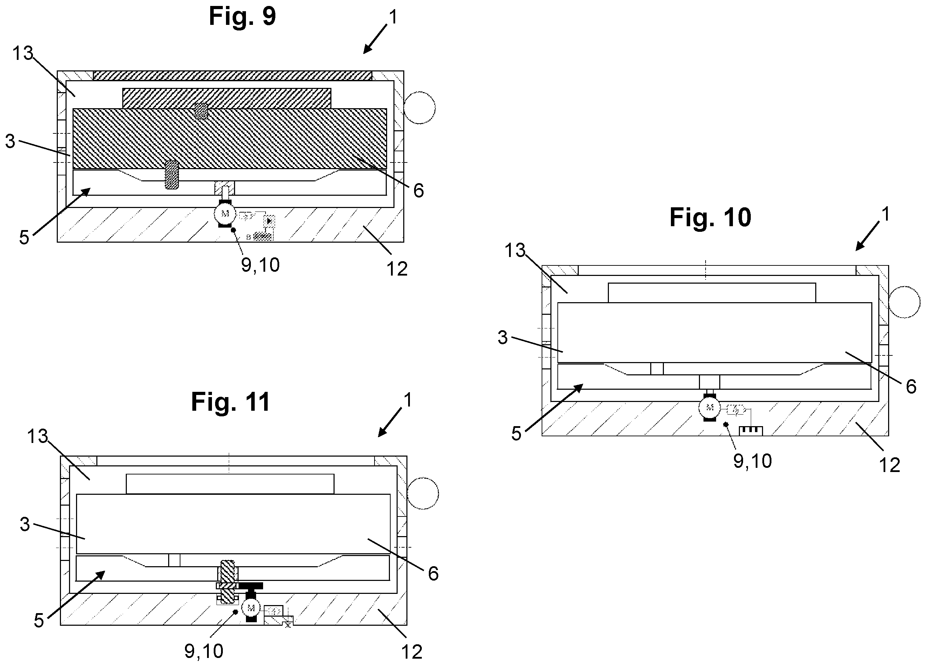

Die Figuren 9 bis 11 zeigen unterschiedliche unterstützende Antriebseinheiten 9 - genauer gesagt: unterschiedliche Elektroantriebe 10 - für die Fördervorrichtung 5, wobei eine Bewegung des Rotors 6 durch die Antriebseinheiten 9 begünstigt und/oder verstärkt werden kann, jedoch die Antriebseinheiten 9 nicht dazu ausgebildet sind, eigenständige Bewegung umsetzt.

So ist in Fig. 9 ein elektrischer Antrieb 10 vorgesehen, um die Bewegung des Rotors 6 zu unterstützen, wobei ein Elektromotor über einen Akkumulator angetrieben wird. Der Akkumulator kann über eine Induktionsschleife aufgeladen werden.

Fig. 10 zeigt einen Elektroantrieb 10 zur Unterstützung der Bewegung des Rotors 6, welcher ebenfalls durch einen Elektromotor umgesetzt ist. Dieser Elektromotor weist ebenfalls einen Akkumulator auf, der über eine entsprechende Schnittstelle und ein Ladegerät aufgeladen werden kann. Diese Schnittstelle kann beispielsweise als USB C-Schnittstelle ausgeführt sein.

Die Ausführungsvariante der Fig. 11 stellt einen Elektroantrieb 10 dar, wobei ein Elektromotor die Bewegung des Rotors 6 unterstützt. Der Elektromotor ist über eine entnehmbare oder austauschbare Batterie angetrieben.

Diese Figuren 9 bis 11 stellen somit eine aktive Antriebseinheit 9 dar, welche dazu ausgebildet ist, eine Bewegung der Fördervorrichtung 5, vorzugsweise des Rotors 6, zu verstärken.

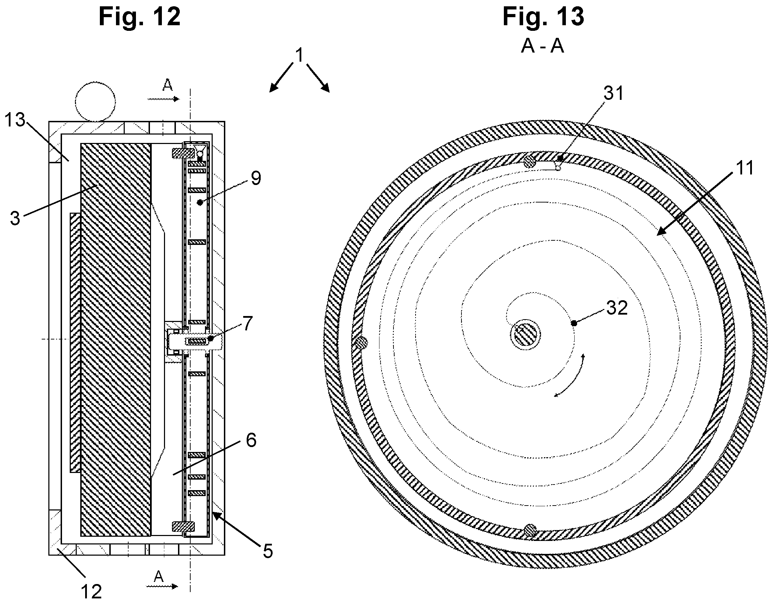

Im Gegensatz dazu zeigen die Figuren 12 und 13 eine passive, mechanische unterstützende Antriebseinheit 9 der Fördervorrichtung 5.

Fig. 12 zeigt wiederum eine geschnittene Seitenansicht der Vorrichtung 1 und Fig. 13 den in Fig. 12 gekennzeichneten Schnitt A-A.

Der Rotor 6 der Fördervorrichtung 5 dieses Ausführungsbeispiels ist wiederum mit der Speichervorrichtung 3 bewegungsschlüssig im Inneren 13 eines Gehäuseelementes 12 verbunden, wobei der Rotor 6 und die Speichervorrichtung 3 bewegungsschlüssig mit der Rotationsachse 7 verbunden sind und somit eine Bewegung um die Rotationsachse 7 ausführen.

Das Gehäuse der Antriebseinheit 9 ist mit dem Gehäuseelement 12 starr verbunden, wohingegen die Rotationsachse 7 beweglich gegenüber dem Gehäuseelement 12 gelagert ist.

Ein (Spiral-) Federelement 32 ist zwischen der Rotationsachse 7 und dem Gehäuse der passiven Antriebseinheit 9 über die Lagerung 31 gelagert. Durch Bewegung der Vorrichtung 1 ergibt sich somit durch eine Unwucht der Rotationsachse 7 (und der damit verbundenen Fördervorrichtung 5, dem Rotor 6 und der Speichervorrichtung 3) eine Bewegung gegenüber dem Gehäuseelement 12.

Diese Bewegung wird durch das Federelement 32 in eine Schwingung umgesetzt, wodurch der rotierende, innere Teil der Fördervorrichtung 5 stets in Schwingung um die Rotationsachse 7 gegenüber dem Gehäuseelement 12 versetzt wird.

Dieses Federelement 32, welches genau genommen als Spiralfeder ausgeführt ist, stellt somit ein Unruh-Spirale-Schwingungssystem 11 dar.

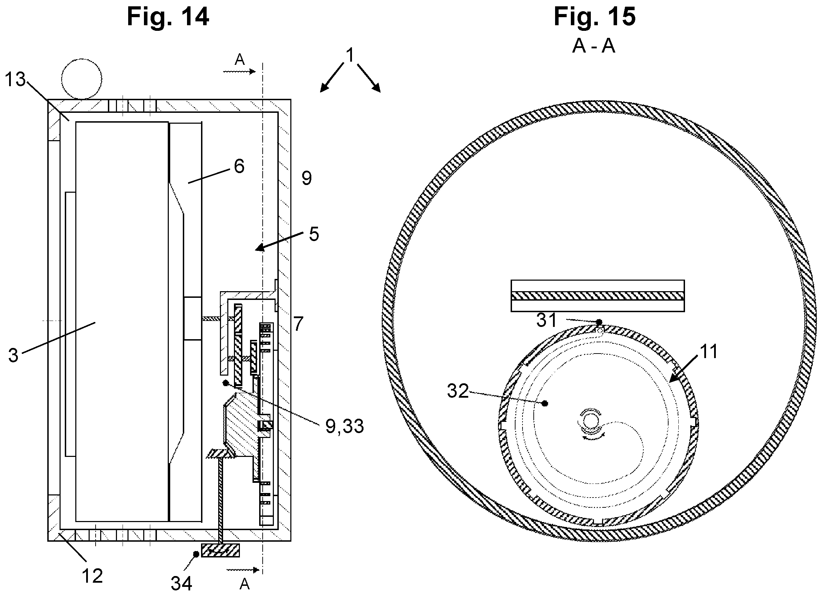

Figuren 14 und 15 zeigen eine weitere Ausgestaltung eines passiven Antriebselementes 9, welche dazu ausgebildet ist, eine Bewegung der Fördervorrichtung 5, vorzugsweise des Rotors 6, zu verstärken. Wiederum ist in diesem Ausführungsbeispiel in Fig. 15 der in Fig. 14 gekennzeichnete Schnitt A-A dargestellt.

Der Aufbau der Vorrichtung 1 ähnelt dem der bereits vorhergehend beschriebenen Varianten, wobei die Speichervorrichtung 3 auf dem Rotor 6 befestigt ist und mit dem Rotor 6 eine Rotationsbewegung zum Fördern des Wirkstoffes 2 ausführen kann.

Der Rotor 6 ist in diesem Ausführungsbeispiel über das mechanische Getriebe 33 unterstützt, welches dem eines Chronographen einer Uhr ähnelt.

Hierbei kann über den Aufzug 34 Energie im Unruh-Spirale-Schwingungssystem 11 gespeichert werden und über das mechanische Getriebe 33 zur Unterstützung einer Bewegung des Rotors 6 an den Rotor 6 und somit die Speichervorrichtung 3 weitergeleitet werden.

Alternativ oder zusätzlich ist auch eine Ausgestaltung denkbar, bei welcher das Gehäuseelement 12 drehbar gelagert ist und durch ein entsprechendes Übersetzungsgetriebe beispielsweise mit dem Unruh-Spirale-Schwingungssystem 11 verbunden ist, wobei durch ein Drehen des Gehäuseelementes 12 (welches als Aufzug 34 dient) durch den Anwender Energie dem Unruh-Spirale-Schwingungssystem 11 zugeführt werden kann.

Das Lagerelement 31, welches das (Spiral-) Federelement 32 am Gehäuse der passiven Antriebseinheit 9 lagert, ist in diesem Ausführungsbeispiel durch eine sogenannte Rutschkupplung ausgebildet, wobei bei einer Überbeanspruchung (beispielsweise durch ein zu hohes Aufziehen durch einen Anwender) das Lagerelement 31 sich löst und in eine andere Lagerposition (Ausnehmung im Gehäuse) verrutscht, um ein Gebrechen des Federelementes 32 zu verhindern.

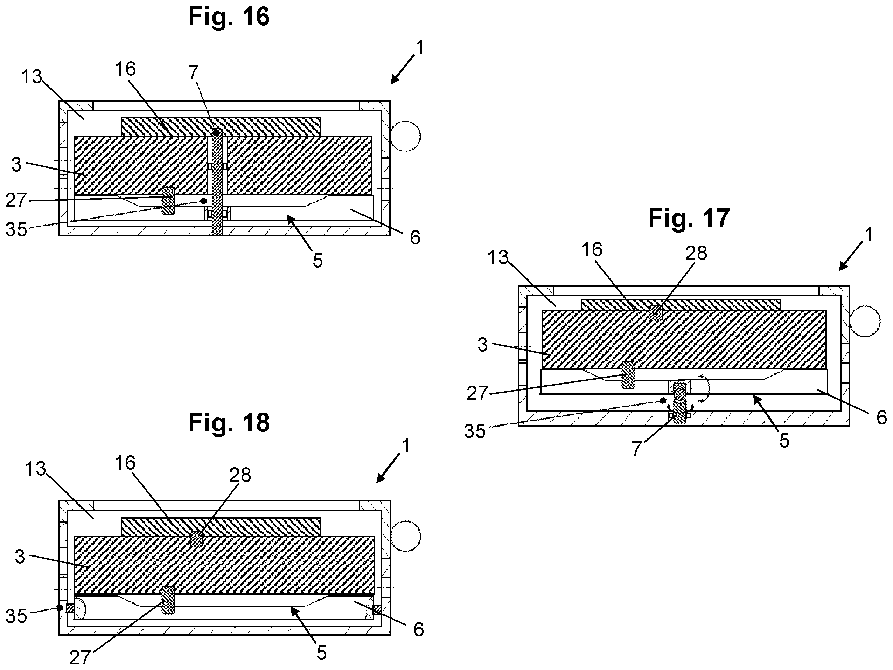

Die Figuren 16 bis 18 zeigen einen Auszug aus verschiedenen möglichen Lagerausführungen der Fördervorrichtung 5.

Die Fördervorrichtung 5 ist wiederum durch einen Rotor 6 ausgebildet, auf welchem bewegungsschlüssig über ein Lager 27 die Speichervorrichtung 3 gelagert ist, wobei wiederum oberhalb der Speichervorrichtung 3 die Anzeigevorrichtung 16 angeordnet ist.

In der Ausführungsvariante der Fig. 16 ist der Rotor 6 gegenüber der Rotationsachse 7 drehbar gelagert. Die Speichervorrichtung ist über das Lager 27 bewegungsschlüssig mit dem Rotor 6 verbunden und führt somit ebenfalls eine Rotationsbewegung gegenüber der Rotationsachse 7 aus. Diese Lagerstellen 35 des Rotors 6 und der Speichervorrichtung 3 können beispielsweise als Rollenlager oder Kugellager ausgebildet sein.

Die Anzeigevorrichtung 16 ist hingegen bewegungsschlüssig mit der Rotationsachse 7 verbunden, wodurch sie starr mit dem Gehäuseelement 12 verbunden ist, sodass die Anzeigevorrichtung 16 stillsteht, während der Rotor 6 zusammen mit der Speichervorrichtung 3 dazu ausgebildet ist, eine Rotationsbewegung im Gehäuseelement 12 auszuführen.

Die Ausführungsvariante der Fig. 17 weist eine Kugel-Lagerstelle 35 (kann auch als Gelenk-Lagerstelle bezeichnet werden) des Rotors 6 gegenüber dem Gehäuseelement 12 auf, wobei nicht nur eine Rotation um die Rotationsachse 7 gestattet wird, sondern auch eine Verkippung, wie durch die Pfeile verdeutlich ist.

Durch eine entsprechende Kugel-Lagerstelle 35 kann über die Fördervorrichtung 5 auch ein Wippen, Drehen, Vibrieren, Pulsieren auf den Wirkstoff 2 ausgeübt werden, welche Bewegungen ein Verteilen, Ausbringen, Verdunsten und/oder Verdampfen des Wirkstoffes 2 begünstigen und/oder fördern kann.

Der Rotor 6 ist wiederum über das Lager 27 bewegungsschlüssig mit der Speichervorrichtung 3 verbunden und die Speichervorrichtung 3 bewegungsschlüssig über das Lager 28 mit der Anzeigevorrichtung 16.

Die Ausführungsvariante der Fig. 18 zeigt eine Lagerstelle 35 des Rotors 6 gegenüber des Gehäuseelementes 12 entlang des Umfangs, wobei wiederum der Rotor 6 über das Lager 27 mit der Speichervorrichtung 3 und die Speichervorrichtung 3 über das Lager 28 mit der Anzeigevorrichtung 16 bewegungsschlüssig verbunden ist.

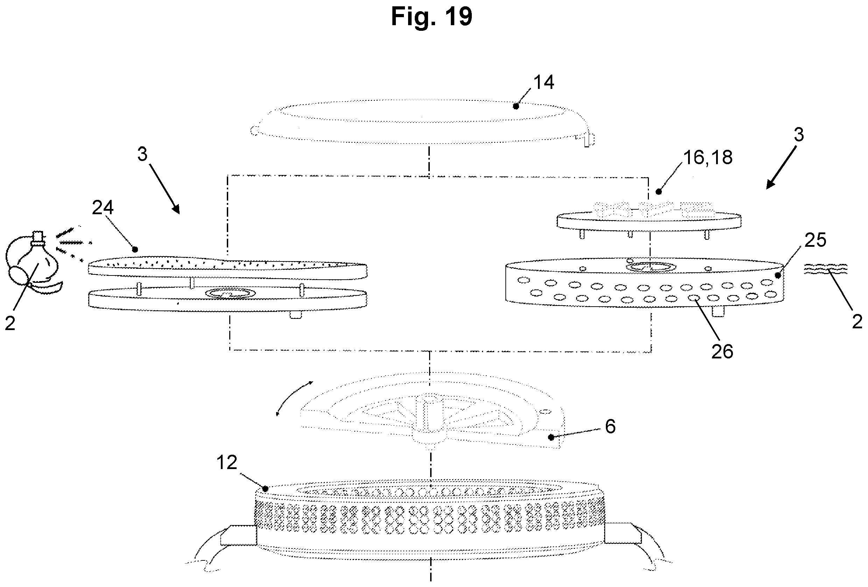

Fig. 19 zeigt eine Anordnung einer Vorrichtung 1 mit einem Wirkstoff 2 in einer Explosionsdarstellung, wobei symbolisch unterschiedliche Speichervorrichtungen 3 für das Gehäuseelement 12 der Vorrichtung 1 gezeigt sind.

So kann in einem Gehäuseelement 12 der Vorrichtung 1 auf einer als Rotor 6 ausgebildeten Fördervorrichtung 5 entweder ein Wirkstoffpad 24 oder ein Trägergefäß 25 angeordnet werden, wobei anschließend das Gehäuseelement 12 durch einen Deckel 14 verschlossen wird.

Das Wirkstoffpad 24 ist dazu ausgebildet, einen Wirkstoff 2 zu speichern, wobei das Wirkstoffpad 24 aus einem den Wirkstoff 2 aufnehmenden Werkstoff ausgebildet ist, wobei der Wirkstoff 2 durch Verdunstung, Verdampfung und/oder mechanischen Einflüsse vom Wirkstoffpad 24 abgebbar ist.

Es kann auch vorgesehen sein, dass das Wirkstoffpad 24 durch einen Anwender mit einem Wirkstoff 2 beladen werden kann. So kann ein Anwender beispielsweise das Wirkstoffpad 24 mit einem handelsüblichen Parfumflacon besprühen, wobei der Wirkstoff 2 (genauer gesagt: das Parfum) vom Anwender aus dem Flacon in das Wirkstoffpad 24 übergeführt wird.

Ein solcher in Frage kommender Werkstoff für ein Wirkstoffpad 24 ist beispielsweise ein gebundenes Gefüge aus Fasern oder Fäden bestehend aus Zellstoff der Baumwolle, synthetischen Fasern, wie Polyester oder Ähnlichem.

Das Trägergefäß 25 ist dazu ausgebildet, einen Wirkstoff 2 in flüssiger, fester und/oder gasförmiger Form zu speichern, wobei der Wirkstoff 2 durch eine Öffnung 27 des Trägergefäßes 25 abgebbar ist.

Auf dem Trägergefäß 25 oder dem Wirkstoffpad 24 kann zusätzlich eine Anzeigevorrichtung 16 (beispielsweise mit einer Werbefläche 18) angeordnet werden.

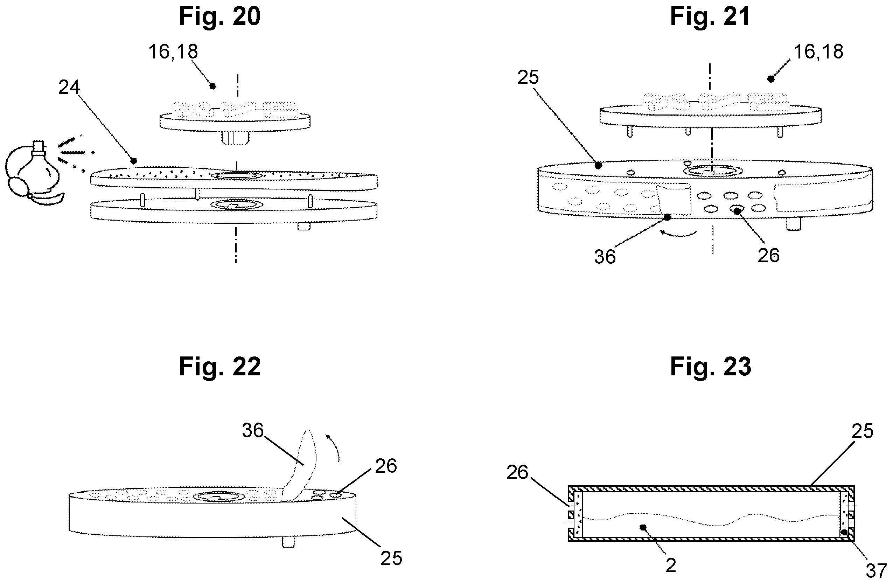

Die Figuren 20 bis 23 zeigen unterschiedliche Ausführungsformen von Speichervorrichtungen 3 für eine Vorrichtung 1.

Die in Fig. 20 dargestellte Speichervorrichtung 3 weist dabei ähnlich wie in Fig. 19 ein Wirkstoffpad 24 auf einem Träger auf, wobei noch zusätzlich eine Anzeigevorrichtung 16 (beispielsweise mit einer Werbefläche 18) befestigt werden kann.

Fig. 21 zeigt ein Trägergefäß 25, welches dazu ausgebildet ist, einen Wirkstoff 2 in flüssiger, fester und/oder gasförmiger Form aufzunehmen, wobei der Wirkstoff 2 über die Öffnungen 26 des Trägergefäßes 25 entweichen kann.

Dieses Trägergefäß 25 weist die Öffnungen 26 entlang eines Umfanges auf, wohingegen die Ausgestaltung der Fig. 22 die Öffnungen 26 auf einer Stirnseite aufweist.

Zum Transport, Lagerung und vor Verwendung des Trägergefäßes 25 können die Öffnungen 26 des Trägergefäßes 25 durch eine Schutzvorrichtung 36 abgedeckt werden.

Diese Schutzvorrichtung 36 der Ausführungsvarianten der Figuren 21 und 22 sind durch abziehbare Folien dargestellt, welche ein Austreten des Wirkstoffes 2 vor Gebrauch der Speichervorrichtung 3 (genauer gesagt: des Trägergefäßes 25) verhindern.

Zusätzlich kann wiederum eine Anzeigevorrichtung 16 (beispielsweise mit einer Werbefläche 18) auf dem Trägergefäß 25 angeordnet werden.

Fig. 23 zeigt einen Schnitt durch ein Trägergefäß 25, wobei die flüssige Lagerung des Wirkstoffes 2 im Inneren des Trägergefäßes 25 ersichtlich ist.

Zwischen dem flüssig vorliegenden Wirkstoff 2 und den Öffnungen 26 des Trägergefäßes 25 ist eine Membran 37 vorgesehen.

Durch diese Membran 37 kann (durch geeignete Werkstoffwahl) eine Abgabe, Verdunstung und/oder Verdampfung des Wirkstoffes 2 über die Öffnungen 26 des Trägergefäßes 25 geregelt werden.

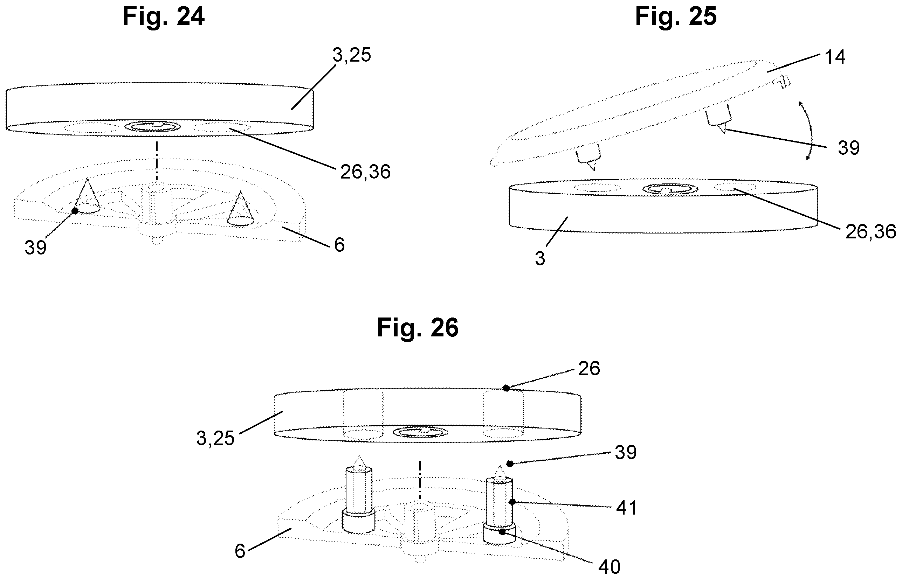

Die Figuren 24 bis 26 zeigen weitere mögliche Ausführungsvarianten einer Anordnung eines Trägergefäßes 25 in einer Vorrichtung 1.

Das Trägergefäß 25 der Ausführungsvarianten der Figuren 24 bis 26 ist jeweils dazu ausgebildet, einen Wirkstoff 2 in flüssiger, fester und/oder gasförmiger Form aufzunehmen.

Das Trägergefäß 25 weist jeweils Öffnungen 26 auf, über welche Öffnungen 26 der Wirkstoff 2 aus dem Inneren des Trägergefäßes 25 austreten kann.

Die Trägergefäße 25 der Figuren 24 bis 26 sind als Speichervorrichtung 3 der Vorrichtung 1 anzusehen.

Für den Transport und/oder die Lagerung dieser Trägergefäße 25 ist jeweils auf der Stirnseite der Trägergefäße (über die Öffnungen 26 der Trägergefäße 25) eine als Folie ausgeführte Schutzvorrichtung 36 angeordnet, um ein ungewolltes Austreten des Wirkstoffes 2 aus dem Trägergefäß 25 zu verhindern.

Um diese Schutzvorrichtung 36 automatisch beim Anordnen des Trägergefäßes 25 in der Vorrichtung 1 zu öffnen, sind in den Figuren 24 bis 26 unterschiedliche Varianten gezeigt.

So kann beispielsweise (wie in Fig. 24 gezeigt) bei einer als Rotor 6 ausgebildeten Fördervorrichtung 5 Dorne 39 zur Aufnahme des Trägergefäßes 25 vorgesehen sein, welche das Trägergefäß 25 einerseits in seiner Lage sichern und andererseits bei der Anordnung die Schutzvorrichtung 36 des Trägergefäßes 25 durchstechen und somit die Öffnungen 26 freigeben.

Diese Dorne 39 können natürlich in einer alternativen Ausführungsvariante (wie durch Fig. 25 gezeigt) auch an einem Deckel 14 des Gehäuseelementes 12 angeordnet sein, wobei bei Aufsetzen des Deckels 14 die Schutzvorrichtung 36 des Trägergefäßes 25 aufgestochen wird.

Jedoch sind auch Ausführungen denkbar, bei denen eine Schutzvorrichtung 36 umfangsseitig am Trägergefäß 25 angeordnet ist, wobei die Dorne 39 entsprechend am Gehäuseelement 12 angeordnet sind, um diese bei der Aufnahme aufzustechen und/oder zu lösen.

Wie in Fig. 26 dargestellt kann jedoch auch vorgesehen sein, dass zur Aufnahme des Trägergefäßes 25 auf einem Rotor 6 Lagerelemente 41 vorgesehen sind, welche auf ihrer oberen Seite Dorne 39 zum Aufstechen der Schutzvorrichtung 36 des Trägergefäßes 25 aufweisen.

Die Lagerelemente 41 können beispielsweise auch aus einem den Wirkstoff 2 aufnehmenden oder transportierenden Werkstoff (ähnlich wie das Wirkstoffpad 24) ausgebildet sein, sodass durch die Lagerelemente 41 der Wirkstoff 2 des Trägergefäßes 25 im Gehäuseelement 12 verteilt, aufgenommen und/oder ein Austritt reguliert werden kann.

An einem unteren Ende der Lagerelemente 41 können des Weiteren Dichtungen 40 vorgesehen sein, um ein Ausrinnen des Trägergefäßes 25 an einer Unterseite des Trägergefäßes 25 zu verhindern.

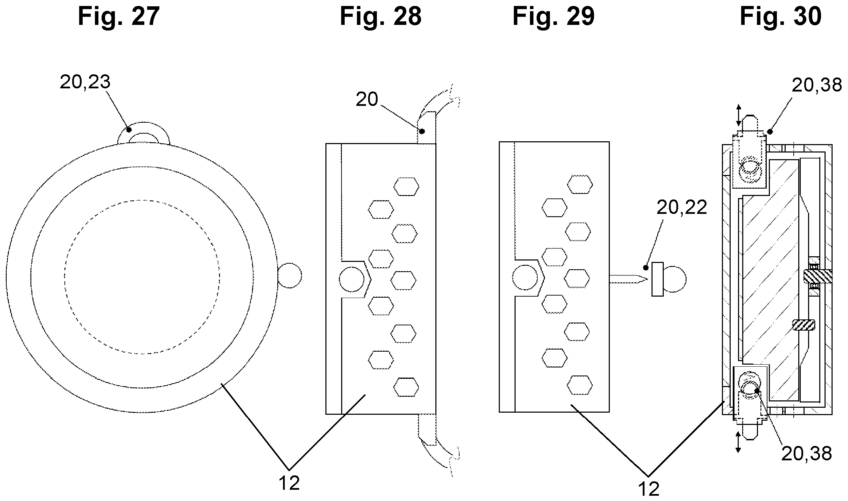

Die Figuren 27 bis 30 zeigen unterschiedliche Ausführungsvarianten einer Haltevorrichtung 20 einer Vorrichtung 1.

So zeigt Fig. 27, dass die Haltevorrichtung 20 einer Vorrichtung 1 als Öse 23 ausgebildet sein kann, durch welche beispielsweise eine Halskette oder ein Armband hindurchgeführt werden kann, um die Vorrichtung 1 am Hals eines Anwenders zu tragen.

Fig. 28 zeigt eine Ausführungsvariante, bei welcher die Vorrichtung 1 eine Haltevorrichtung 20 zum Halten eines Armbandes 21 aufweist, sodass die Vorrichtung 1 wie eine Uhr am Handgelenk eines Anwenders tragbar ist.

Fig. 29 zeigt eine Ausführungsvariante einer Haltevorrichtung 20 als Anstecknadel 22, sodass die Vorrichtung 1 an einer beliebigen Stelle eines Kleidungsstückes eines Anwenders über die Anstecknadel 22 ähnlich einer Brosche angeheftet werden kann. Die in Fig. 29 gezeigte Haltevorrichtung 20 kann durch die Anstecknadel 22 auch dazu dienen die Vorrichtung 1 als Ohrring zu tragen.

Fig. 30 zeigt ein Ausführungsbeispiel, bei welchem die Haltevorrichtung 20 eine Klemmvorrichtungen 38 aufweist.

Diese Klemmvorrichtungen sind dazu ausgebildet, die Vorrichtung 1 an einer Schnur zu halten, wobei die Schnur durch die Klemmvorrichtung 38 hindurchgeführt wird und anschließend ein Bolzen über eine Feder die Schnur in der Klemmvorrichtung 38 verklemmt und somit die Vorrichtung 1 an der Schnur hält.

Dementsprechend kann die Vorrichtung 1 beispielsweise auch an Schnürsenkeln eines Schuhs oder an einer Kordel eines Kapuzenpullovers befestigt werden.

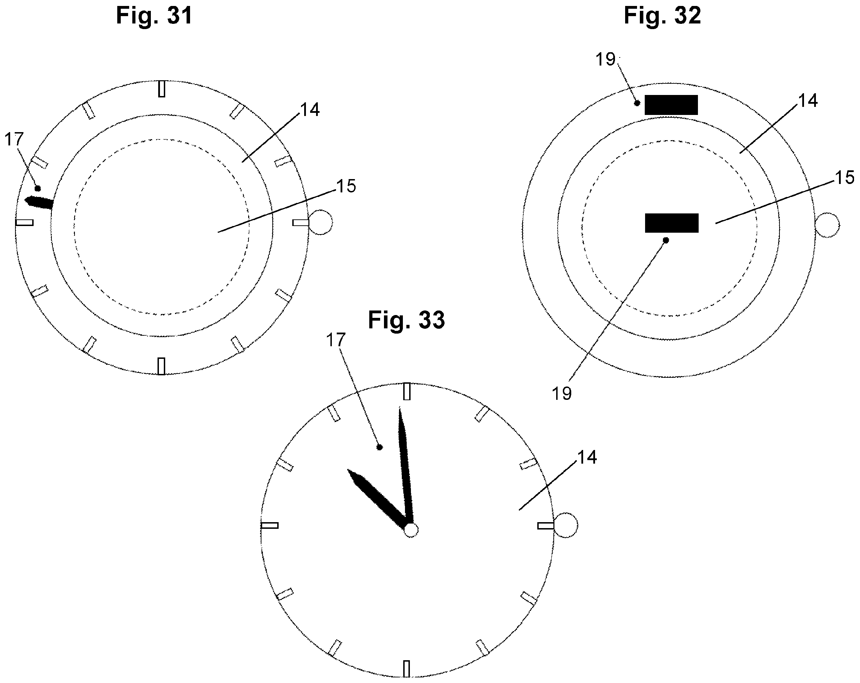

Die Figuren 31 bis 33 zeigen unterschiedliche Ausführungsvarianten einer Anzeigevorrichtung 16 einer Vorrichtung 1.

In Fig. 31 ist eine Ausführungsvariante ersichtlich, bei welcher der Deckel 14 der Vorrichtung 1 mit einem zentrischen, transparenten Bereich 15 ausgebildet ist, sodass das Innere 13 des Gehäuses 12 für einen Anwender ersichtlich ist.

In einem äußeren Bereich des Deckels 14 ist ein weiterer transparenter Bereich 15 vorgesehen, in welchen als Anzeigevorrichtung 16 eine Uhrzeitanzeige 17 mit Zeiger angeordnet ist, sodass lediglich die Spitze des Uhrzeigers zu erkennen ist und eine Uhrzeit ablesbar ist.

Im Ausführungsbeispiel der Fig. 32 ist im inneren transparenten Bereich 15 des Deckels 14 sowie im äußeren Bereich des Deckels 14 ein Display 19 angeordnet, wobei über das Display 19 beispielsweise eine Uhrzeit, ein Datum oder andere Texte und Symbole (ähnlich wie bei einer Smartwatch) angezeigt werden können.

Dieses Display 19 kann auch an anderen Stellen des Deckels 14 oder des Gehäuses 12 angeordnet sein und sich großflächig über den gesamten Deckel 14 - vorzugsweise den gesamten Deckel 14 - oder Gehäuse 12 erstrecken.

Weitere Inhalte, welche auf einem solchen Display 19 beispielsweise angezeigt werden können, sind neben üblichen Smartwach Apps auch Gesundheitsinhalte (wie beispielsweise der Puls des Trägers oder dessen Sauerstoffsättigung im Blut). Eine entsprechend notwendige Sensorik kann beispielsweise in oder am Gehäuse 12 angeordnet sein.

Das Ausführungsbeispiel der Fig. 33 zeigt, dass die Anzeigevorrichtung 16 am Deckel 14 des Gehäuseelementes 12 als Uhrzeitanzeige 17 ähnlich zu einer Armbanduhr ausgeführt sein kann, wobei sich zwei Zeiger (Stunden- und Minutenzeiger) zur Anzeige einer Uhrzeit bewegen.

Die gezeigten Lagervarianten und Befestigungsmethoden der einzelnen Elemente der Vorrichtung 1 der Ausführungsbeispiele der Figuren sind rein beispielhaft zu verstehen und können natürlich durch adäquate dem Fachmann bekannte lagersichernde Varianten (beispielsweise kraft-, form- oder stoffschlüssige Verbindungen) ersetzt werden.

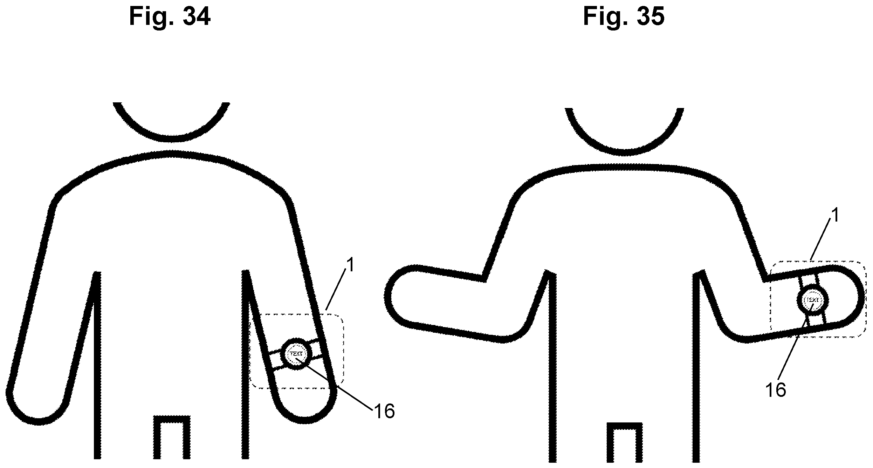

Die Figuren 34 und 35 verdeutlichen den Vorteil, wenn die Anzeigevorrichtung 16 (hier durch das Wort "TEXT" angedeutet) mit dem wenigstens einen Rotor 6 der Vorrichtung 1 drehfest verbunden ist, wobei durch den Rotor 6 und eine wirkende Erdanziehung die Anzeigevorrichtung 16 stets horizontal ausgerichtet ist.

Im dargestellten Beispiel der Figuren 34 und 35 wird dies durch die Anwendung der Vorrichtung 1 verdeutlicht, wobei die Vorrichtung ähnlich einer Armbanduhr am Körper eines Anwenders getragen wird, wobei in verschiedenen Stellungen des Armes des Anwenders (wie durch die Fig. 34 und 35 verdeutlicht) die Vorrichtung eine unterschiedliche Relativlage einnimmt.

Dies kann jedoch durch den Rotor 6 und seine asymmetrische Gewichtsverteilung gegenüber der Rotationsachse 7 ausgeglichen werden, da sich durch die wirkende Erdanziehung sich der Rotor 6 stets horizontal ausrichten wird.

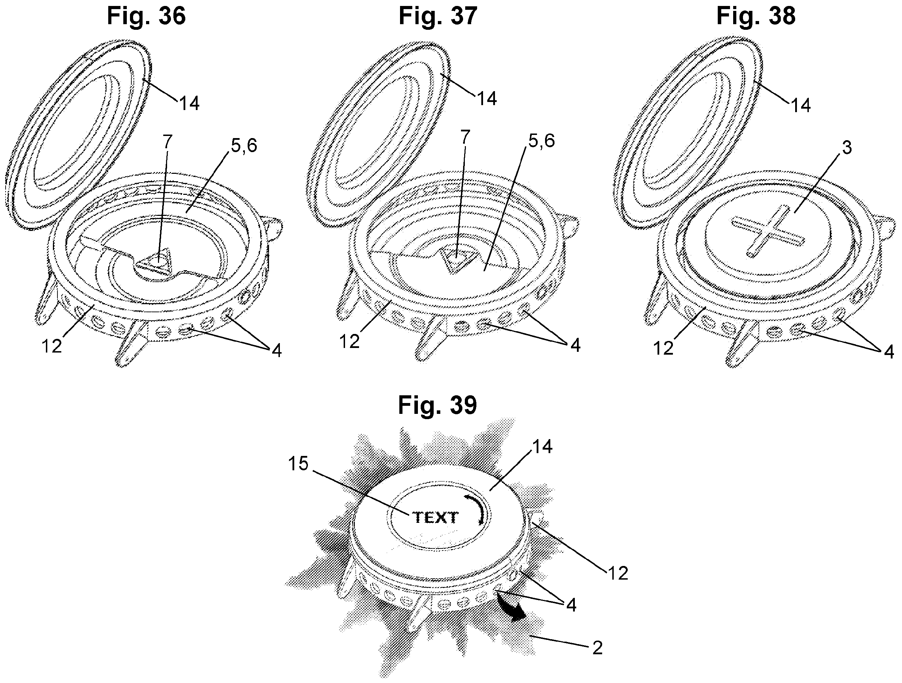

Die Figuren 36 bis 42 zeigen ein weiteres Ausführungsbeispiel einer tragbaren Vorrichtung 1 zur Abgabe eines Wirkstoffes 2.

Die Vorrichtung 1 dieser Ausführungsvariante weist ein Gehäuseelement 12 auf, welches ähnlich einer Armbanduhr aufgebaut ist, wobei über die gezeigten Befestigungselemente ähnlich einer Armbanduhr ein Armband 21 mit dem Gehäuse 12 verbunden werden könnte.

Des Weiteren ist am Gehäuseelement 12 ein öffenbarer Deckel 14 angeordnet, welcher über eine Drehachse gegenüber dem Gehäuseelement 12 zwischen einer geöffneten und einer geschlossenen Stellung verklappt werden kann. Eine geöffnete Stellung ist beispielsweise durch die Figuren 36 bis 38 gezeigt und eine geschlossene Stellung durch die Figur 39.

Im Inneren des Gehäuseelements 12 ist die Fördervorrichtung 5 vorgesehen.

Diese Fördervorrichtung 5 ist durch den Rotor 6 ausgebildet, welcher um die Rotationsachse 7 rotierbar mit dem Gehäuseelement 12 verbunden ist, wie durch die Figuren 36 und 37 verdeutlicht wird.

Auf dem Rotor 6 kann die Speichervorrichtung 3 angeordnet werden, wobei diese Speichervorrichtung 3 dieses Ausführungsbeispiels drehfest, lösbar mit dem Rotor 6 verbindbar ist (wie im späteren noch detaillierter beschrieben wird).

Ein solches Aufsetzen der Speichervorrichtung 3 auf den Rotor 6 ist durch die Figur 38 gezeigt.

Nach Anordnen der Speichervorrichtung 3 auf dem Rotor 6 kann der Deckel 14 geschlossen werden, wobei es durch eine anschließende Bewegung der Vorrichtung 1 zu einer Bewegung des Rotors 6 und der mit dem Rotor 6 bewegungsschlüssig verbundenen Speichervorrichtung 3 kommt, wodurch Wirkstoff 2 aus der Speichervorrichtung 3 über die Ausbringöffnungen 4 förderbar ist (wie durch die Fig. 39 angedeutet).

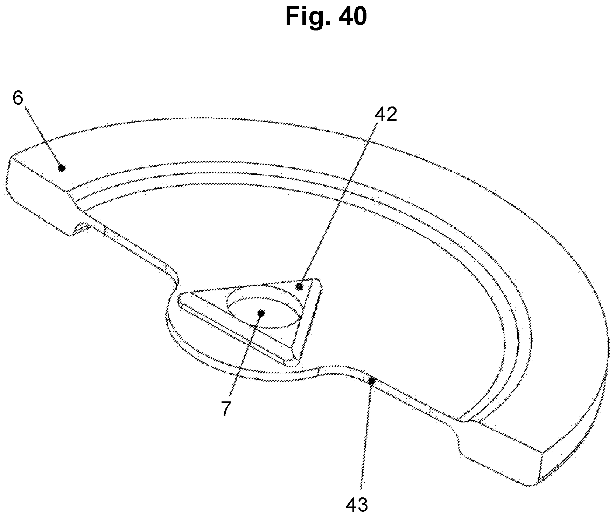

Fig. 40 zeigt den Rotor 6 der Ausführungsvariante der Figuren 36 bis 42 in Isolation und Detailansicht, wobei zu erkennen ist, wie der Rotor 6 durch seine geometrische Ausgestaltung eine Unwucht bezüglich ihrer Rotationsachse 7 (in anderen Worten: eine Gewichtsasymmetrie) aufweist.

Gelagert oder gehalten wird der Rotor 6 durch eine in diesen Figuren nicht ersichtliche Lagerung an der Unterseite des Rotors 6.

Weiters weist der Rotor 6 eine Verbindungsvorrichtung 42 auf, welche dazu ausgebildet ist, die Speichervorrichtung 3 aufzunehmen. Diese Verbindungsvorrichtung 42 ist durch ihre geometrische Gestalt dermaßen ausgebildet, dass eine formschlüssige Verbindung zwischen der Speichervorrichtung 3 und dem Rotor 6 hergestellt wird.

Durch diese formschlüssige Verbindung kann vermieden werden, dass eine Speichervorrichtung 3 nicht frei gegenüber dem Rotor 6 platziert werden kann, sondern eine definierte Position zu dieser aufweist.

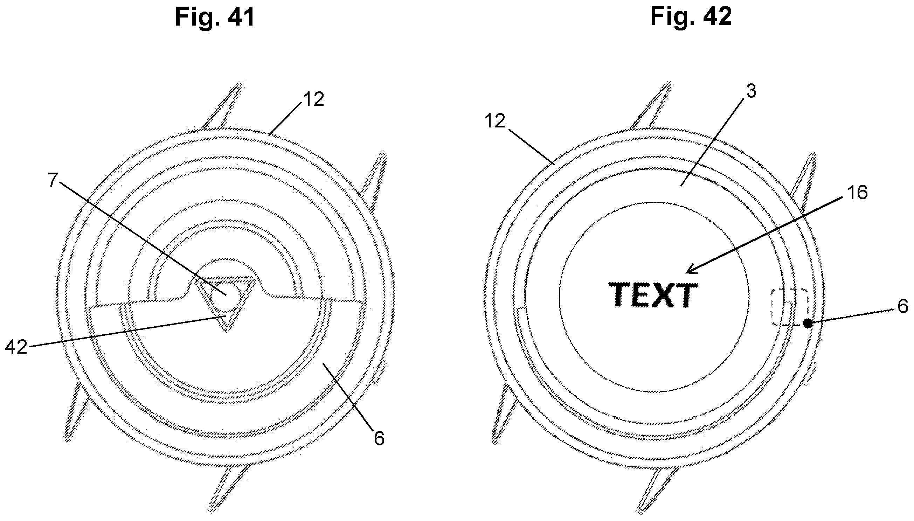

Dieses Vorlegen einer definierten Position zwischen dem Rotor 6 und der Speichervorrichtung 3 ist vor allem dann von Vorteil, wenn die Speichervorrichtung 3 eine Anzeigevorrichtung 16 aufweist, welche gemeinsam mit dem Rotor 6 ausgerichtet werden soll (siehe hierzu noch Fig. 42).

Um die Verbindung zwischen Speichervorrichtung 3 und Rotor 6 zu verstärken, kann beispielsweise vorgesehen sein, dass ein zusätzlicher Magnet vorgesehen ist, um die Speichervorrichtung 3 am Rotor 6 zu halten.

Weiters ist durch Fig. 40 ersichtlich, dass eine Kippkante 43 des Rotors 6 beispielsweise entlang ihrer Längserstreckung eine von einer Geraden abweichende Form aufweisen kann. So weist die Kippkante des gezeigten Ausführungsbeispiels eine Kurve im Bereich der zentralen Rotationsachse 7 auf.

Die Figuren 41 und 42 verdeutlichen nochmals den Anwendungsfall, in welchem es vorteilhaft ist, dass die Speichervorrichtung 3 zum Rotor 6 eine definierte Position einnimmt, sodass eine Anzeigevorrichtung 16 (in der Fig. 41 als "TEXT" dargestellt) stets durch Unterzuhilfenahme des Rotors 6 eine horizontale Ausrichtung einnimmt.

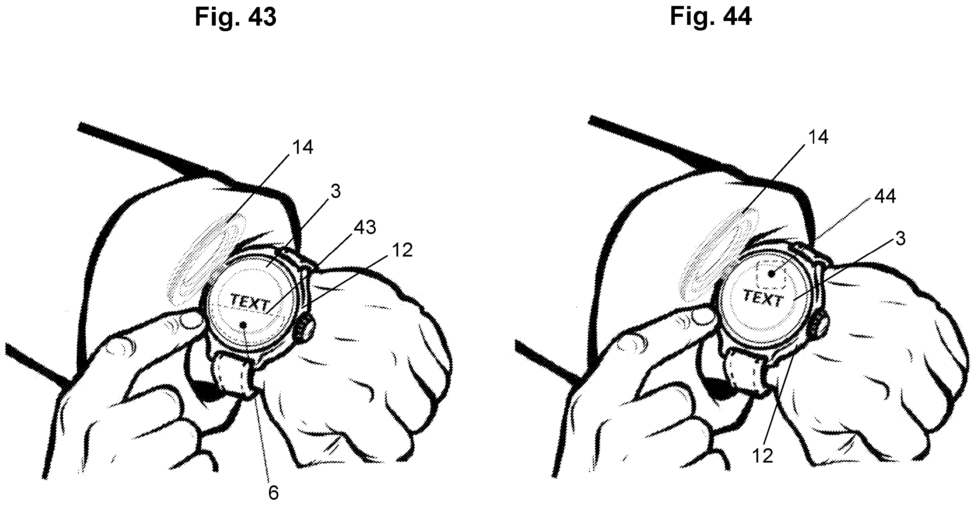

Die Figuren 43 bis 49 zeigen ein weiteres Ausführungsbeispiel einer Vorrichtung 1 zur Abgabe eines Wirkstoffes 2 dar, welche Vorrichtung 1 wiederum ähnlich einer Armbanduhr an einem Armgelenk eines Anwenders tragbar ist, wie durch die Figuren 43 und 44 ersichtlich ist.

Dieses Ausführungsbeispiel stellt eine besonders bedienerfreundliche Möglichkeit dar, die Speichervorrichtung 3 aus dem Gehäuse 12 zu entnehmen, wobei zunächst der Deckel 14 der Vorrichtung 1 geöffnet wird und anschließend auf einen Bereich 44 (strichliert in Fig. 44 dargestellt) durch einen Bediener beispielsweise über den Finger eine Druckkraft aufgebracht werden kann.

Durch Aufbringen der Druckkraft verkippt nun die Speichervorrichtung 3 über eine Kippkante 43, welche durch den Rotor 6 selbst umgesetzt wird (wie beispielsweise strichliert durch die Fig. 43 ersichtlich ist).

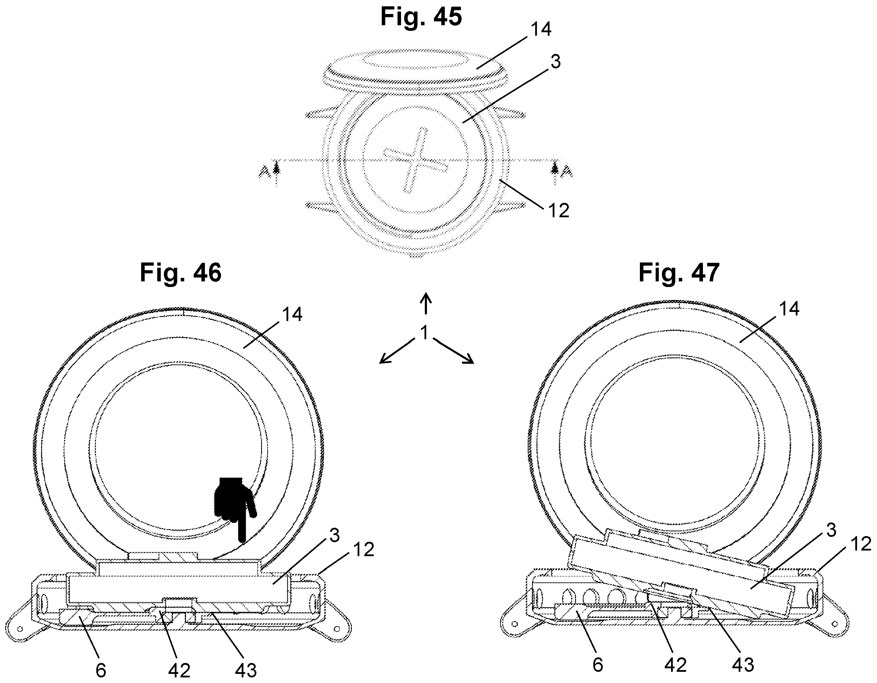

Die Figuren 45 bis 47 zeigen dieses Entnehmen nochmals detaillierter in einer Schnittdarstellung, wobei in Fig. 45 der durch die Figuren 46 und 47 dargestellte Schnitt A-A angedeutet ist.

So ist in Fig. 46 ein verbundener Zustand der Speichervorrichtung 3 mit dem Rotor 6 zu erkennen, wobei der Rotor 6 drehbar im Gehäuseelement 12 angeordnet ist.

Die Speichervorrichtung 3 ist über die Verbindungsvorrichtung 42 mit dem Rotor 6 verbunden.

Wenn nun einseitig durch den Anwender eine Druckkraft auf die Speichervorrichtung 3 ausgeübt wird (wie symbolisch in Fig. 46 gezeigt ist), verkippt die Speichervorrichtung 3 über die Kippkante 43 des Rotors 6, wodurch die Verbindungsvorrichtung 42 zwischen Speichervorrichtung 3 und Rotor 6 gelöst wird (wie durch Fig .47 gezeigt).

Zusätzlich bewegt sich die Speichervorrichtung 3 in einem gekippten Zustand zum Gehäuseelement 12, wodurch es für einen Anwender gestattet wird, bequem die Speichervorrichtung 3 zu entnehmen.

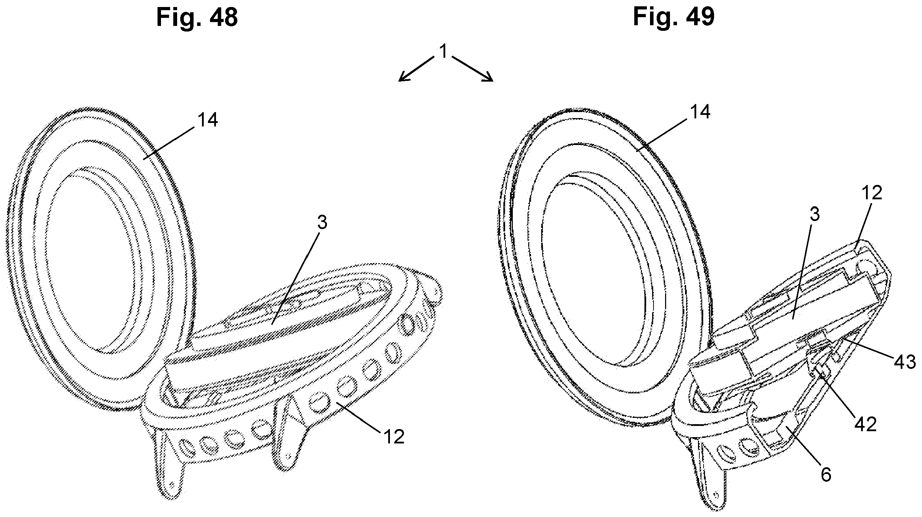

Dieser verkippte Zustand der Speichervorrichtung 3 gegenüber dem Rotor 6 ist auch nochmals durch die Figuren 48 und 49 gezeigt, wobei Fig. 49 wiederum eine Schnittdarstellung der Fig. 48 zeigt.

### Bezugszeichenliste:

- 1: Vorrichtung
- 2: Wirkstoff
- 3: Speichervorrichtung
- 4: Ausbringöffnung
- 5: Fördervorrichtung
- 6: Rotor
- 7: Rotationsachse
- 8: Strömungsleiteinrichtung
- 9: Antriebseinheit
- 10: Elektroantrieb
- 11: Unruh-Spirale-Schwingsystem
- 12: Gehäuseelement
- 13: Inneres des Gehäuseelementes
- 14: Deckel
- 15: transparenter Teilbereich
- 16: Anzeigevorrichtung
- 17: Uhrzeitanzeige
- 18: Werbefläche
- 19: Display
- 20: Haltevorrichtung
- 21: Armband
- 22: Anstecknadel
- 23: Öse
- 24: Wirkstoffpad
- 25: Trägergefäß
- 26: Öffnung des Trägergefäßes
- 27: Lager der Speichervorrichtung
- 28: Lager der Anzeigevorrichtung
- 29: Mechanismus
- 30: Verschlusselement
- 31: Lagerung
- 32: Federelement
- 33: mechanisches Getriebe
- 34: Aufzug
- 35: Lagerstelle
- 36: Schutzvorrichtung
- 37: Membran
- 38: Klemmvorrichtung
- 39: Dorn
- 40: Dichtung
- 41: Lagerelement
- 42: Verbindungsvorrichtung
- 43: Kippkante
- 44: Bereich

## Patentansprüche

1. Am Körper eines Anwenders tragbare Vorrichtung (1) zur Abgabe, Verdunstung und/oder Verdampfung eines Wirkstoffes (2), umfassend,
- wenigstens eine Speichervorrichtung (3) zur Speicherung des Wirkstoffes (2),
- wenigstens einer Ausbringöffnung (4), durch welche der Wirkstoff (2) aus der wenigstens einen Speichervorrichtung (3) in eine Umgebung der Vorrichtung (1) entweichen kann,
wobei eine mit bewegbaren mechanischen Mitteln vorgesehene Fördervorrichtung (5) zur Erzeugung einer Luftbewegung vorgesehen ist, durch welche Wirkstoff (2) durch die wenigstens einen Ausbringöffnung (4) förderbar ist, wobei die bewegbaren mechanischen Mittel durch wenigstens einen Rotor (6) umgesetzt sind, welcher um wenigstens eine Rotationsachse (7) drehbar gelagert ist, **dadurch gekennzeichnet, dass** der wenigstens eine Rotor (6), wenigstens eine Kippkante (43) aufweist, welche Kippkante (43) dazu ausgebildet ist, dass die wenigstens eine Speichervorrichtung (3) zur Entnahme aus der tragbaren Vorrichtung (1) bei einer asymmetrischen Krafteinwirkung auf die wenigstens eine Speichervorrichtung (3) über die Kippkante (43) verkippt.

2. Tragbare Vorrichtung nach Anspruch 1, wobei die Fördervorrichtung (5) dazu ausgebildet ist, vorzugsweise direkt, die wenigstens eine Speichervorrichtung (3) zur Speicherung des Wirkstoffes (2) aufzunehmen.

3. Tragbare Vorrichtung nach dem vorhergehenden Anspruch, wobei die wenigstens eine Speichervorrichtung (3) mit der Fördervorrichtung (5) durch wenigstens eine Verbindungsvorrichtung (42) lösbar befestigbar ist.

4. Tragbare Vorrichtung nach wenigstens einem der vorhergehenden Ansprüche, wobei der wenigstens eine Rotor (6) die wenigstens eine Verbindungsvorrichtung (42) und/oder die wenigstens eine Kippkante (43) aufweist und/oder ausbildet.

5. Tragbare Vorrichtung nach wenigstens einem der vorhergehenden Ansprüche, wobei die wenigstens eine Speichervorrichtung (3) ein Gehäuseelement (12) aufweist, welches Gehäuseelement (12) dazu ausgebildet ist, den Wirkstoff (2) aufzunehmen, vorzugsweise wobei
- die wenigstens eine Ausbringöffnung (4) zumindest eine Öffnung - besonders bevorzugt Bohrung und/oder Durchgangsloch - im Gehäuseelement (12) aufweist, welche das Innere (13) des Gehäuseelementes (12) mit der Umgebung verbindet, und/oder
- das Gehäuseelement (12) wenigstens einen transparenten Teilebereich (15) aufweist - besonders bevorzugt in Form eines transparenten Deckels (14) - mittels welchem das Innere (13) des Gehäuseelement (12) durch einen Anwender ersichtlich ist, und/oder
- die Fördervorrichtung (5) im Inneren (13) des Gehäuseelementes (12) angeordnet ist.

6. Tragbare Vorrichtung nach wenigstens einem der vorhergehenden Ansprüche, wobei die Vorrichtung (1) einen öffenbaren Deckel (14) aufweist, wobei durch den öffenbaren Deckel (14) der Wirkstoff (2) der wenigstens einen Speichervorrichtung (3) und/oder die Speichervorrichtung (3) zuführbar und/oder entnehmbar ist.

7. Tragbare Vorrichtung nach wenigstens einem der vorhergehenden Ansprüche, wobei die Vorrichtung (1) zumindest als Teil einer tragbaren Uhr - vorzugsweise einer Armbanduhr - und/oder eines Schmuckstücks ausgebildet ist.

8. Tragbare Vorrichtung nach wenigstens einem der vorhergehenden Ansprüche, wobei die Vorrichtung (1) wenigstens eine Anzeigevorrichtung (16) aufweist, wobei die wenigstens eine Anzeigevorrichtung vorzugsweise
- eine Uhrzeitanzeige (17) und/oder
- eine Datumsanzeige und/oder
- eine Gravur und/oder
- eine Werbefläche (18) und/oder
- ein Display (19) und/oder
- ein NFT-Kunstwerk und/oder
- einen QR-Code und/oder
- ein Symbol
aufweist.

9. Tragbare Vorrichtung nach wenigstens einem der vorhergehenden Ansprüche, wobei die Vorrichtung (1) eine Haltevorrichtung (20) - vorzugsweise umfassend ein Armband (21) und/oder eine Anstecknadel (22) und/oder eine Öse (23) und/oder eine Klemmvorrichtung (38) - aufweist, wobei die Vorrichtung (1) mittels der Haltevorrichtung (20) an einem Körper eines Anwenders befestigbar und/oder tragbar ist.

10. Tragbare Vorrichtung nach wenigstens einem der vorhergehenden Ansprüche, wobei die Vorrichtung (1) eine zylindrische Außenform aufweist, dessen Durchmesser vorzugsweise mit einer geringeren Abmessung ausgebildet ist als eine Längserstreckung.

11. Tragbare Vorrichtung nach wenigstens einem der vorhergehenden Ansprüche, wobei die wenigstens eine Fördervorrichtung (5) wenigstens eine aktive Antriebseinheit (9) - vorzugsweise einen Elektroantrieb (10) - und/oder wenigstens eine passive Antriebseinheit (9) - vorzugsweise ein Unruh-Spirale-Schwingsystem (11) - aufweist, welche dazu ausgebildet ist, eine Bewegung der Fördervorrichtung (5), vorzugsweise des wenigstens einen Rotors (6), zu verstärken.

12. Tragbare Vorrichtung nach wenigstens einem der vorhergehenden Ansprüche, wobei der wenigstens eine Rotor (6) quer zur Rotationsachse (7) einen Vorsprung und/oder eine Strömungsleiteinrichtung (8) und/oder eine Finne zur Förderung der Luftbewegung aufweist.

13. Anordnung einer tragbaren Vorrichtung (1) nach wenigstens einem der vorhergehenden Ansprüche und zumindest einem Wirkstoff (2), vorzugsweise einem Parfum, wobei der zumindest eine Wirkstoff (2) in der wenigstens einen Speichervorrichtung (3) angeordnet ist.

14. Anordnung nach Anspruch 13, wobei der zumindest ein Wirkstoff (2)
- in einem Wirkstoffpad (24) gespeichert ist, welches aus einem den zumindest einen Wirkstoff (2) aufnehmenden Werkstoff ausgebildet ist, wobei der zumindest eine Wirkstoff (2) durch Verdunstung, Verdampfung und/oder mechanische Einflüsse vom Wirkstoffpad (24) abgebbar ist, und/oder
- in einem Trägergefäß (25) zur flüssigen, festen und/oder gasförmigen Aufnahme des zumindest einen Wirkstoffes (2) gespeichert ist, wobei der zumindest eine Wirkstoff (2) durch eine Öffnung (27) im Trägergefäß (25) abgebbar ist,
wobei das Wirkstoffpad (24) und/oder das Trägergefäß (25) in der wenigstens einen Speichervorrichtung (5) angeordnet ist.

15. Verwendung
- eines Wirkstoffpads (24), in welchem zumindest ein Wirkstoff (2) gespeichert ist, wobei der zumindest eine Wirkstoff (2) durch Verdunstung, Verdampfung und/oder mechanische Einflüsse vom Wirkstoffpad (24) abgebbar ist, und/oder
- eines Trägergefäßes (25), welches dazu ausgebildet ist, zumindest einen flüssigen, festen und/oder gasförmigen Wirkstoffes (2) aufzunehmen, wobei der zumindest eine Wirkstoff (2) durch eine Öffnung (27) im Trägergefäß (25) abgebbar ist,
in einer tragbaren Vorrichtung nach wenigstens einem der Ansprüche 1 bis 12 und/oder einer Anordnung nach Anspruch 13 oder 14.

## Claims

1. A wearable device (1) that can be worn on the body of a user for dispensing, evaporating and/or vaporizing an active substance (2), comprising,
- at least one storage device (3) for storing the active substance (2),
- at least one discharge opening (4) through which the active substance (2) can escape from the at least one storage device (3) into an environment of the device (1),
wherein a conveying device (5) provided with movable mechanical means is provided for generating an air movement, by means of which active substance (2) can be conveyed through the at least one dispensing opening (4), wherein the movable mechanical means are implemented by at least one rotor (6) which is mounted so as to be rotatable about at least one axis of rotation (7), **characterized in that** the at least one rotor (6) has at least one tilting edge (43), which tilting edge (43) is designed such that the at least one storage device (3) tilts over the tilting edge (43) for removal from the wearable device (1) when an asymmetric force is applied to the at least one storage device (3).

2. The wearable device according to claim 1, wherein the conveying device (5) is designed to receive, preferably directly, the at least one storage device (3) for storing the active substance (2).

3. The wearable device according to the preceding claim, wherein the at least one storage device (3) is detachably fastenable to the conveying device (5) by at least one connecting device (42).

4. The wearable device according to at least one of the preceding claims, wherein the at least one rotor (6) has and/or forms the at least one connecting device (42) and/or the at least one tilting edge (43).

5. The wearable device according to at least one of the preceding claims, wherein the at least one storage device (3) has a housing element (12), which housing element (12) is designed to receive the active substance (2), preferably wherein
- the at least one dispensing opening (4) has at least one opening - particularly preferably a bore and/or through-hole - in the housing element (12) which connects the interior (13) of the housing element (12) with the environment, and/or
- the housing element (12) has at least one transparent part region (15) - particularly preferably in the form of a transparent cover (14) - by means of which the interior (13) of the housing element (12) can be seen by a user, and/or
- the conveying device (5) is arranged in the interior (13) of the housing element (12).

6. The wearable device according to at least one of the preceding claims, wherein the device (1) has an openable cover (14), wherein the active substance (2) of the at least one storage device (3) and/or the storage device (3) can be supplied and/or removed through the openable cover (14).

7. The wearable device according to at least one of the preceding claims, wherein the device (1) is designed at least as part of a portable watch - preferably a wristwatch - and/or a piece of jewelry.

8. The wearable device according to at least one of the preceding claims, wherein the device (1) comprises at least one display device (16), wherein the at least one display device preferably has
- a time display (17) and/or
- a date display and/or
- an engraving and/or
- an advertising space (18) and/or
- a display (19) and/or
- an NFT artwork and/or
- a QR code and/or
- a symbol.

9. The wearable device according to at least one of the preceding claims, wherein the device (1) has a holding device (20) - preferably comprising a bracelet (21) and/or a pin (22) and/or an eyelet (23) and/or a clamping device (38) - wherein the device (1) can be fastened and/or worn on a body of a user by means of the holding device (20).

10. The wearable device according to at least one of the preceding claims, wherein the device (1) has a cylindrical outer shape, the diameter of which is preferably formed with a smaller dimension than a longitudinal extent.

11. The wearable device according to at least one of the preceding claims, wherein the at least one conveying device (5) has at least one active drive unit (9) - preferably an electric drive (10) - and/or at least one passive drive unit (9) - preferably a balance-spring oscillation system (11) - which is designed to increase a movement of the conveying device (5), preferably of the at least one rotor (6).

12. The wearable device according to at least one of the preceding claims, wherein the at least one rotor (6) has a projection and/or a flow guide device (8) and/or a fin transversely to the rotation axis (7) for promoting air movement.

13. An arrangement of a wearable device (1) according to at least one of the preceding claims and at least one active substance (2), preferably a perfume, wherein the at least one active substance (2) is arranged in the at least one storage device (3).

14. The arrangement according to claim 13, wherein the at least one active substance (2)
- is stored in an active substance pad (24) which is made of a material which absorbs the at least one active substance (2), wherein the at least one active substance (2) can be dispensed from the active substance pad (24) by evaporation, vaporization and/or mechanical influences, and/or
- is stored in a carrier vessel (25) for liquid, solid and/or gaseous holding of the at least one active substance (2), wherein the at least one active substance (2) can be dispensed through an opening (27) in the carrier vessel (25),
wherein the active substance pad (24) and/or the carrier vessel (25) are arranged in the at least one storage device (5).

15. Use
- of an active substance pad (24) in which at least one active substance (2) is stored, wherein the at least one active substance (2) can be dispensed from the active substance pad (24) by evaporation, vaporization and/or mechanical influences, and/or
- of a carrier vessel (25) which is designed to hold at least one liquid, solid and/or gaseous active substance (2), wherein the at least one active substance (2) can be dispensed through an opening (27) in the carrier vessel (25),
in a wearable device according to at least one of claims 1 to 12 and/or an arrangement according to claim 13 or 14.

## Revendications

1. Dispositif (1) portatif sur le corps d'un utilisateur destiné à délivrer, évaporer et/ou vaporiser une substance active (2), comprenant,
- au moins un dispositif de stockage (3) pour stocker la substance active (2),
- au moins une ouverture de distribution (4) par laquelle la substance active (2) peut s'échapper de l'au moins un dispositif de stockage (3) dans un environnement du dispositif (1),
dans lequel un dispositif de convoyage (5) prévu avec des moyens mécaniques mobiles est prévu pour générer un mouvement d'air par lequel la substance active (2) peut être convoyée à travers l'au moins une ouverture de distribution (4), dans lequel les moyens mécaniques mobiles sont mis en œuvre par au moins un rotor (6) qui est monté de manière à pouvoir tourner autour d'au moins un axe de rotation (7), **caractérisé en ce que** l'au moins un rotor (6) présente au moins un bord basculant (43), lequel bord basculant (43) est réalisé pour que l'au moins un dispositif de stockage (3) bascule au-delà du bord de basculement (43) pour être retiré du dispositif portatif (1) en cas d'application d'une force asymétrique sur l'au moins un dispositif de stockage (3).

2. Dispositif portatif selon la revendication 1, dans lequel le dispositif de convoyage (5) est réalisé pour recevoir de préférence directement l'au moins un dispositif de stockage (3) pour stocker la substance active (2).

3. Dispositif portatif selon la revendication précédente, dans lequel l'au moins un dispositif de stockage (3) peut être fixé de manière amovible au dispositif de convoyage (5) par au moins un dispositif de liaison (42).

4. Dispositif portatif selon au moins l'une quelconque des revendications précédentes, dans lequel l'au moins un rotor (6) présente et/ou forme l'au moins un dispositif de liaison (42) et/ou l'au moins un bord de basculement (43).

5. Dispositif portatif selon au moins l'une quelconque des revendications précédentes, dans lequel l'au moins un dispositif de stockage (3) présente un élément de boîtier (12), lequel élément de boîtier (12) est réalisé pour recevoir la substance active (2), de préférence dans lequel
- l'au moins une ouverture de distribution (4) présente au moins une ouverture - de manière particulièrement préférée un alésage et/ou un trou de passage - dans l'élément de boîtier (12), qui relie l'intérieur (13) de l'élément de boîtier (12) à l'environnement, et/ou
- l'élément de boîtier (12) présente au moins une zone partielle transparente (15) - de manière particulièrement préférée sous la forme d'un couvercle transparent (14) - au moyen de laquelle l'intérieur (13) de l'élément de boîtier (12) est visible par un utilisateur, et/ou
- le dispositif de convoyage (5) est disposé à l'intérieur (13) de l'élément de boîtier (12).

6. Dispositif portatif selon au moins l'une quelconque des revendications précédentes, dans lequel le dispositif (1) présente un couvercle ouvrant (14), dans lequel la substance active (2) peut être amenée à l'au moins un dispositif de stockage (3) et/ou le dispositif de stockage (3) peut être retiré par le couvercle (14) ouvrant.

7. Dispositif portatif selon au moins l'une quelconque des revendications précédentes, dans lequel le dispositif (1) est réalisé au moins en tant que partie d'une montre portable - de préférence d'une montre-bracelet - et/ou d'un bijou.

8. Dispositif portatif selon au moins l'une quelconque des revendications précédentes, dans lequel le dispositif (1) présente au moins un dispositif d'affichage (16), dans lequel l'au moins un dispositif d'affichage présente de préférence
- un affichage de l'heure (17) et/ou
- un affichage de la date et/ou
- une gravure et/ou
- une surface publicitaire (18) et/ou
- un écran (19) et/ou
- une œuvre d'art NFT et/ou
- un code QR et/ou
- un symbole

9. Dispositif portatif selon au moins l'une quelconque des revendications précédentes, dans lequel le dispositif (1) présente un dispositif de maintien (20) - comprenant de préférence un bracelet (21) et/ou une aiguille (22) et/ou un œillet (23) et/ou un dispositif de serrage (38) -, dans lequel le dispositif (1) peut être fixé et/ou supporté sur le corps d'un utilisateur au moyen du dispositif de maintien (20).

10. Dispositif portatif selon au moins l'une quelconque des revendications précédentes, dans lequel le dispositif (1) présente une forme extérieure cylindrique dont le diamètre est de préférence réalisé avec une dimension inférieure à une extension longitudinale.

11. Dispositif portatif selon au moins l'une quelconque des revendications précédentes, dans lequel l'au moins un dispositif de convoyage (5) présente au moins une unité d'entraînement (9) active - de préférence un entraînement électrique (10) - et/ou au moins une unité d'entraînement (9) passive - de préférence un système oscillant (11) à spirale agitée - qui est réalisé pour amplifier un mouvement du dispositif de convoyage (5), de préférence de l'au moins un rotor (6).

12. Dispositif portatif selon au moins l'une quelconque des revendications précédentes, dans lequel l'au moins un rotor (6) présente une saillie et/ou un dispositif de guidage d'écoulement (8) et/ou une ailette pour favoriser le mouvement d'air transversalement à l'axe de rotation (7).

13. Ensemble d'un dispositif portatif (1) selon au moins l'une quelconque des revendications précédentes et d'au moins une substance active (2), de préférence un parfum, dans lequel l'au moins une substance active (2) est disposée dans l'au moins un dispositif de stockage (3).

14. Ensemble selon la revendication 13, dans lequel l'au moins une substance active (2)
- est stockée dans un tampon de substance active (24) qui est réalisé à partir d'un matériau recevant l'au moins une substance active (2), dans lequel l'au moins une substance active (2) peut être libérée par évaporation, vaporisation et/ou des influences mécaniques du tampon de substance active (24), et/ou
- est stockée dans un récipient de support (25) pour l'absorption liquide, solide et/ou gazeuse de l'au moins une substance active (2), dans lequel l'au moins une substance active (2) peut être libérée par une ouverture (27) dans le récipient de support (25),
dans lequel le tampon de substance active (24) et/ou le récipient de support (25) sont disposés dans l'au moins un dispositif de stockage (5).

15. Utilisation
- d'un tampon de substance active (24) dans lequel au moins une substance active (2) est stockée, dans laquelle l'au moins une substance active (2) peut être libérée par évaporation, vaporisation et/ou des influences mécaniques du tampon de substance active (24), et/ou
- d'un récipient de support (25), qui est réalisé pour recevoir au moins une substance active (2) liquide, solide et/ou gazeuse, dans laquelle l'au moins une substance active (2) peut être libérée par une ouverture (27) dans le récipient de support (25),
dans un dispositif portatif selon au moins l'une quelconque des revendications 1 à 12 et/ou un ensemble selon la revendication 13 ou 14.
